(19) Europäisches Patentamt
European Patent Office
Office européen des brevets

(11) Veröffentlichungsnummer: **0 329 606**
**A2**

(12) # EUROPÄISCHE PATENTANMELDUNG

(21) Anmeldenummer: **89810094.6**

(22) Anmeldetag: **03.02.89**

(51) Int. Cl.⁴: **C 07 D 471/18**
C 07 F 9/65, A 61 K 31/495,
A 61 K 31/675, C 07 K 5/00,
C 07 K 7/00
//C12P17/18,(C07D471/18,
241:00,221:00,221:00)

(30) Priorität: **12.02.88 CH 514/88 12.02.88 CH 515/88**

(43) Veröffentlichungstag der Anmeldung:
**23.08.89 Patentblatt 89/34**

(84) Benannte Vertragsstaaten:
**AT BE CH DE ES FR GB GR IT LI LU NL SE**

(71) Anmelder: **CIBA-GEIGY AG**
**Klybeckstrasse 141**
**CH-4002 Basel (CH)**

**Gesellschaft für Biotechnologische Forschung mbH (GBF)**
**Mascheroder Weg 1**
**D-3300 Braunschweig-Stöckheim (DE)**

(72) Erfinder: **Reichenbach, Hans, Prof. Dr.**
**Platanenstrasse 35**
**D-3340 Wolfenbüttel (DE)**

**Trowitzsch-Kienast, Wolfram, Dr.**
**Am Hasengarten 8**
**D-3300 Braunschweig (DE)**

**Gerth, Klaus, Dr.**
**Am Butterbusch 17**
**D-3300 Braunschweig (DE)**

**Irschik, Herbert, Dr.**
**Masurenweg 15**
**D-3340 Wolfenbüttel (DE)**

**Kunze, Brigitte, Dr.**
**Hinter Aegidien 5**
**D-3300 Braunschweig (DE)**

**Augustiniak, Hermann, Dr.**
**In den Lindendöhren 19**
**D-3340 Wolfenbüttel (DE)**

**Bedorf, Norbert, Dr.**
**Krukenbergstrasse 4**
**D-3308 Königslutter (DE)**

**Jansen, Rolf, Dr.**
**Falkenbergstrasse 14**
**D-3300 Braunschweig (DE)**

**Höfle, Gerhard, Prof. Dr.**
**Alter Weg 12a**
**D-3300 Braunschweig (DE)**

**Steinmetz, Heinrich**
**Magdalenenweg 15**
**D-3320 Hildesheim-Sorsum (DE)**

Der Anmelder hat eine Erklärung nach Regel 28 (4) EPÜ (Herausgabe einer Probe nur an einen Sachverständigen) eingereicht. Eingangsnummer(n) der Hinterlegung(en): NCIB 12268

(54) **Neue Antibiotika aus Myxococcus.**

(57) Die Erfindung betrifft neue polycyclische Alkaloide der Formel

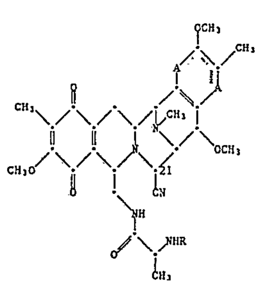

(I),

worin R Wasserstoff, Acyl, Sulfonyl, Sulfo oder Phospho, und A C=O oder gegebenenfalls acyliertes C-OH bedeuten, wobei die gestrichelte Linie eine C=C-Doppelbindung an der Stelle der mittleren Bindung, wenn A C=O ist, oder an der Stelle der beiden äusseren Bindungen, wenn A gegebenenfalls acyliertes C-OH ist, symbolisiert, ein Verfahren zur Herstellung dieser Verbindungen durch Cyanidierung und gegebenenfalls Acylierung oder Sulfonylierung in beliebiger Reihenfolge des Fermentationsprodukts eines Mikroorganismus der Spezies Myxococcus xanthus, pharmazeutische Präparate, die die neuen Verbindungen enthalten, und die Verwendung der neuen

EP 0 329 606 A2

Verbindungen als Antibiotika und als tumorhemmende Mittel und zur Herstellung von pharmazeutischen Präparaten. Die Erfindung betrifft auch die neuen Zwischenprodukte der Formel I, worin CN durch OH ersetzt ist und R durch Carboxy oder verestertes Carboxy substituiertes Acyl, Sulfo oder Phospho ist, die ebenfalls pharmazeutich verwendbar sind.

**Beschreibung**

**Neue Antibiotika aus Myxococcus**

Die Erfindung betrifft neue polycyclische Alkaloide mit antibiotischen Eigenschaften, ein Verfahren zur Herstellung dieser Verbindungen durch Cyanidierung und gegebenenfalls Acylierung oder Sulfonylierung des Fermentationsprodukts eines Mikroorganismus der Spezies Myxococcus xanthus, pharmazeutische Präparate, die die neuen Verbindungen enthalten, und die Verwendung der neuen Verbindungen als Antibiotika und als tumorhemmende Mittel und zur Herstellung von pharmazeutischen Präparaten.

Die Erfindung betrifft insbesondere Verbindungen der Formel

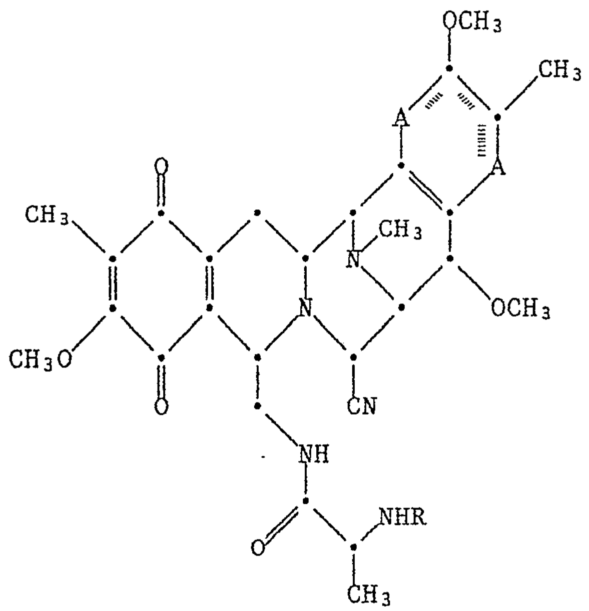

(I),

worin R Wasserstoff, Acyl, Sulfonyl, Sulfo oder Phospho und A C=O oder gegebenenfalls acyliertes C-OH bedeuten, wobei die gestrichelte Linie eine C=C-Doppelbindung an der Stelle der mittleren Bindung, wenn A C=O ist, oder an der Stelle der beiden äusseren Bindungen, wenn A gegebenenfalls acyliertes C-OH ist, symbolisiert, und Salze solcher Verbindungen, insbesondere pharmazeutisch verwendbare Salze.

Die erfindungsgemässen Verbindungen der Formel I weisen nahe Verwandtschaft mit den aus Streptomyces lavendulae isolierten Verbindungen auf, die Saframycine genannt werden. In U.S. Patent 4 248 863 ist beispielsweise die fermentative Herstellung der Saframycine A, B, C, D und E, in U.S. Patent 4 372 947 die Herstellung von Saframycin S beschrieben. Die Struktur der bekannten Saframycine ist in T. Arai, Antimicrobial Agents and Chemotherapy 28, 5 (1985) angeführt. Demgegenüber unterscheiden sich die erfindungsgemässen Verbindungen der Formel I von den vorbekannten Saframycinen zumindest durch den Alanyl-Rest in der Seitenkette, an dessen Stelle in den vorbekannten Saframycinen der Acylrest der Brenztraubensäure oder ein anderer Acylrest steht, ferner zusätzlich auch noch durch die Methoxygruppe am überbrückten bicyclischen Kern. Saframycin-Derivate mit einem Alanyl-Rest in der Seitenkette sind in der Europäischen Patentanmeldung EP 173 649 beschrieben, unterscheiden sich aber von den erfindungsgemässen Verbindungen durch eine fehlende Methoxygruppe.

In der Europäischen Patentanmeldung EP 55 299 sind die Antibiotica Y-16482 $\alpha$ und $\beta$ beschrieben, die durch Fermentation eines Stammes von Pseudomonas fluorescens erhalten werden. Diese Verbindungen, auch Safracine genannt, weisen nach Y. Ikeda et al., J. Antibiotics 36, 1284 (1983) nahe Verwandtschaft mit den Saframycinen und damit auch mit den erfindungsgemässen Verbindungen der Formel I auf. Die Safracine unterscheiden sich jedoch von den vorliegenden neuen Verbindungen durch die Bedeutung von A (eine der Gruppen A ist C-H) und das Fehlen der Methoxygruppe am überbrückten bicyclischen Kern.

Weitere nah verwandte Verbindungen können aus dem Schwamm Reniera sp. isoliert werden. Diese von J.M. Frinke und D.J. Faulkner, J. Am. Chem. Soc. 104, 265 (1982) Renieramycine genannten Verbindungen unterscheiden sich von den erfindungsgemässen Verbindungen der Formel I zumindest durch die Seitenkette, die statt Alanylaminomethyl in den Renieramycinen (Z)-2-Methyl-2-butenoyloxymethyl (ein Angelicasäureester) ist.

Die Konfiguration an den chiralen Zentren der erfindungsgemässen Verbindungen der Formel I ist nicht mit Sicherheit bekannt. Hinweise für die relative Konfiguration an den verschiedenen chiralen Zentren ergeben sich allerdings aus dem "Nuclear Overhauser Effect" in Protonen-Kernresonanzspektren und aus der Analyse

des bei der Hydrolyse freigesetzten Alanins. In Analogie zur bekannten, durch Röntgenstrukturanalyse bestimmten absoluten Konfiguration von Saframycin C (T. Arai et al., Tetrahedron Letters 1979, 2355) und von bromiertem Safracin A (I. Ueda et al., Acta Cryst. C 40, 1578 (1984)) ist anzunehmen, dass die erfindungsgemässen Verbindungen die räumliche Struktur gemäss Formel

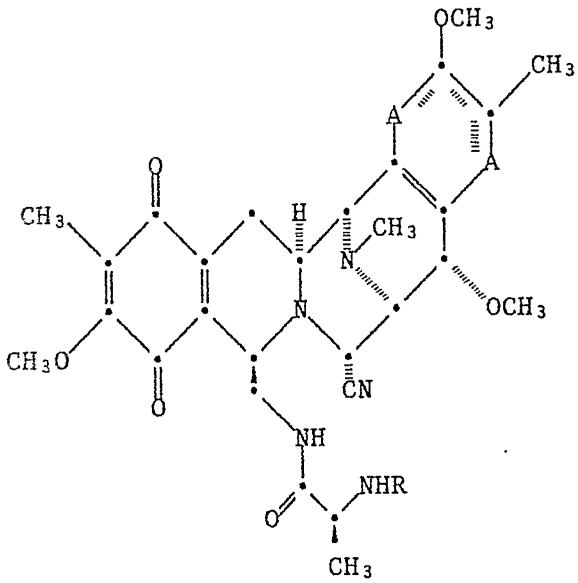

(Ia)

aufweisen.

In Verbindungen der Formel I und Ia bedeutet R Wasserstoff, Acyl, Sulfonyl, Sulfo oder Phospho. Acyl R ist beispielsweise die Acylgruppe einer Carbonsäure, eines Kohlensäurehalbesters, einer Carbaminsäure oder einer Thiocarbaminsäure mit bis zu 20 Kohlenstoffatomen, beispielsweise $C_1$-$C_{20}$-Alkanoyl, durch Hydroxy, verethertes oder verestertes Hydroxy, Amino, acyliertes Amino, Carboxy, amidiertes oder verestertes Carboxy, Oxo und/oder Halogen substituiertes $C_2$-$C_7$-Alkanoyl, Aroyl, $C_1$-$C_7$-Alkoxycarbonyl, $C_1$-$C_7$-Alkylaminocarbonyl, $C_1$-$C_7$-Alkylaminothiocarbonyl, Arylaminocarbonyl oder Arylaminothiocarbonyl. Acyl $R^2$ kann auch der Rest eines Polypeptids, z.B. eines Polypeptids aus den üblichen in der Natur vorkommenden Aminosäuren, vorzugsweise aus 2-200 Aminosäuren, sein.

Sulfonyl R ist beispielsweise ein Rest einer Sulfonsäure mit bis zu 20 Kohlenstoffatomen, z.B. $C_1$-$C_7$-Alkansulfonyl oder Arylsulfonyl, oder unsubstituiertes oder substituiertes Aminosulfonyl. Sulfo bedeutet den Schwefelsäurerest $-SO_3H$. Phospho bedeutet den Phosphorsäurerest $-PO_3H_2$.

$C_1$-$C_{20}$-Alkanoyl ist vorzugsweise $C_1$-$C_7$-Alkanoyl, beispielsweise Formyl, Acetyl, Propionyl, n-Butyryl, 2-Methylpropionyl, n-Pentanoyl, 2,2-Dimethylpropionyl, 2-Methylbutyryl, 3-Methylbutyryl oder n-Hexanoyl, oder geradkettiges $C_8$-$C_{20}$-Alkanoyl mit einer geraden Anzahl Kohlenstoffatome, beispielsweise n-Octanoyl, n-Decanoyl, n-Dodecanoyl, n-Tetradecanoyl, n-Hexadecanoyl, n-Octadecanoyl oder n-Icosanoyl.

Durch Hydroxy substituiertes $C_2$-$C_7$-Alkanoyl ist beispielsweise Glykoloyl, Glyceroyl oder Lactoyl, wobei die Hydroxygruppe verethert, z.B. mit $C_1$-$C_4$-Alkyl, wie Methyl, Ethyl oder Isopropyl, oder mit Aryl-$C_1$-$C_4$-alkyl, wie Benzyl, oder verestert sein kann, z.B. mit $C_1$-$C_7$-Alkanoyl, wie Formyl, Acetyl, Propionyl oder Pivaloyl.

Durch Amino substituiertes $C_2$-$C_7$-Alkanoyl ist z.B. Glycyl, Alanyl, β-Alanyl, Valyl, Norvalyl, Leucyl, Isoleucyl, γ-Aminobutyryl, α-γ-Diaminobutyryl, Lysyl oder Ornithyl. Acyl $R^2$ umfasst auch Acylreste anderer Aminosäuren, z.B. Acylreste der natürlich vorkommenden α-Aminosäuren, wie Prolyl, Arginyl, Cysteinyl, Aspartyl, β-Aspartyl, Asparaginyl, Glutamyl, γ-Glutamyl, Glutaminyl, Phenylalanyl, Tyrosyl, Seryl, Histidinyl, Methionyl, Tryptophyl oder Threonyl, entweder in der natürlichen L-Form oder in der unnatürlichen D-Form. Die Aminogruppe(n) der genannten Aminosäuren können acyliert sein, beispielsweise durch eine der vorstehend oder nachstehend genannten Acylgruppen z.B. durch $C_1$-$C_4$-Alkoxycarbonyl, wie tert-Butoxycarbonyl, oder durch Aryl-$C_1$-$C_4$-alkoxycarbonyl, wie Benzyloxycarbonyl.

Durch Carboxy substituiertes $C_2$-$C_7$-Alkanoyl ist beispielsweise Malonyl, Succinyl, Glutaryl oder Adipoyl mit je einer freien Carboxygruppe. Diese Carboxygruppe kann auch amidiert oder verestert sein, z.B. mit einem $C_1$-$C_7$-Alkanol, wie Methanol, Ethanol oder tert-Butanol, oder mit einem Aryl-$C_1$-$C_4$-alkanol, wie Benzylalkohol.

Durch Oxo substituiertes $C_2$-$C_7$-Alkanoyl ist beispielsweise Glyoxyloyl oder Pyruvoyl. Durch Halo substituiertes $C_2$-$C_7$-Alkanoyl ist beispielsweise Trifluoracetyl, Mono-, Di- oder Trichloracetyl.

Aroyl ist beispielsweise Benzoyl oder 1- oder 2-Naphthoyl, worin der Phenyl- oder Naphthylring durch Nitro, Amino, Halogen, beispielsweise Chlor oder Brom, Hydroxy, $C_1$-$C_4$-Alkoxy, beispielsweise Methoxy, Carboxy, Carbamoyl und/oder verestertes Carboxy, z.B. $C_1$-$C_4$-Alkoxycarbonyl, wie Methoxycarbonyl, substituiert sein

kann, beispielsweise 4-Nitrobenzoyl, 3,5-Dinitrobenzoyl, Anthraniloyl, 2,6-Dichlorbenzoyl, Salicyloyl, Galloyl, 2-, 3- oder 4-Anisoyl, Phthaloyl, Terephthaloyl, 2-Carbamoylbenzoyl oder 2-Methoxycarbonylbenzoyl.

$C_1$-$C_7$-Alkoxy-carbonyl ist beispielsweise Methoxy-, Ethoxy-, n-Propoxy-, Isopropoxy-, n-Butoxy-, Isobutoxy-oder tert-Butoxy-carbonyl.

$C_1$-$C_7$-Alkylaminocarbonyl und $C_1$-$C_7$-Alkylaminothiocarbonyl sind beispielsweise Methylamino-carbonyl oder -thiocarbonyl, Ethylamino-carbonyl oder -thiocarbonyl, n- oder tert-Butylamino-carbonyl oder -thiocarbonyl, oder n-Hexylamino-carbonyl oder -thiocarbonyl. Arylaminocarbonyl und Arylthiocarbonyl sind beispielsweise Phenylamino-carbonyl oder -thiocarbonyl, 1-oder 2-Naphthylamino-carbonyl oder -thiocarbonyl, oder Phenylaminocarbonyl oder -thiocarbonyl, worin Phenyl durch $C_1$-$C_4$-Alkyl, z.B. Methyl, wie 2- oder 4-Methyl oder 3,5-Dimethyl, Nitro, z.B. 4-Nitro, Halogen, z.B. Chlor oder Brom, wie 4-Chlor, 4-Brom oder 2,6-Dichlor, oder $C_1$-$C_4$-Alkoxy, z.B. Methoxy, wie 2- oder 4-Methoxy, substituiert sein kann.

$C_1$-$C_7$-Alkansulfonyl ist beispielsweise Methansulfonyl, Ethansulfonyl oder n-Butansulfonyl. Arylsulfonyl ist beispielsweise Benzolsulfonyl, 1- oder 2-Naphthalinsulfonyl oder substituiertes Benzolsulfonyl, worin der Substituent $C_1$-$C_4$-Alkyl, z.B. Methyl oder Isopropyl, Nitro, Halogen, z.B. Chlor oder Brom, oder $C_1$-$C_4$-Alkoxy, z.B. Methoxy, sein kann, z.B. wie in p-Toluolsulfonyl, p-Nitrobenzolsulfonyl, 2,4-Dinitrobenzolsulfonyl, p-Brombenzolsulfonyl oder p-Methoxybenzolsulfonyl.

Substituiertes Aminosulfonyl ist beispielsweise durch eine oder zwei $C_1$-$C_7$-Alkylgruppen und/oder Aryl-$C_1$-$C_4$-Alkylgruppen substituiert. In substituiertem Aminosulfonyl kann Amino auch Teil eines fünf- oder sechsgliedrigen Heterocyclus enthaltend ein bis zwei Stickstoffatome und gewünschtenfalls ein Sauerstoff- oder Schwefelatom sein. Beispiele für substituiertes Aminosulfonyl sind Methylaminosulfonyl, Ethylaminosulfonyl, n- oder tert-Butylaminosulfonyl, Dimethylaminosulfonyl, Diethylaminosulfonyl, Benzylaminosulfonyl, Pyrrolidinosulfonyl, Piperidinosulfonyl, 4-Methylpyridazinylsulfonyl, Morpholinosulfonyl oder Thiomorpholinosulfonyl.

Bevorzugte Reste Acyl R sind $C_1$-$C_7$-Alkanoyl, z.B. Formyl, Acetyl, Propionyl, n-Butyryl, 2,2-Dimethylpropionyl oder n-Hexanoyl, durch gegebenenfalls acyliertes Amino und/oder gegebenenfalls verestertes Carboxy substituiertes $C_2$-$C_7$-Alkanoyl, z.B. Glycyl, Alanyl, β-Alanyl, Valyl, γ-Aminobutyryl, oder Succinyl, Glutaryl oder Adipoyl mit einer freien Carboxygruppe, Aspartyl, β-Aspartyl, Glutamyl oder γ-Glutamyl, oder einer der genannten Reste, worin die Aminogruppe durch Benzyloxycarbonyl acyliert und/oder worin die Carboxygruppe durch Methyl oder Benzyl verestert ist, Aroyl, z.B. Benzoyl, Phthaloyl oder 2-Methoxycarbonylbenzoyl, Arylaminocarbonyl, z.B. Phenylaminocarbonyl oder Arylaminothiocarbonyl, z.B. Phenylaminothiocarbonyl. Bevorzugter Rest Sulfonyl R ist Arylsulfonyl, z.B. Benzolsulfonyl, p-Toluolsulfonyl oder p-Nitrobenzolsulfonyl.

Der Acylrest in der Gruppe A mit der Bedeutung acyliertes C-OH hat dieselbe Bedeutung wie Acyl R. Bevorzugter Acylrest in der Gruppe A ist beispielsweise $C_1$-$C_7$-Alkanoyl, z.B. Formyl, Acetyl, Propionyl oder 2,2-Dimethylpropionyl, oder durch Carboxy oder verestertes Carboxy substituiertes $C_2$-$C_7$-Alkanoyl, z.B. Succinyl, Glutaryl oder durch Methyl verestertes Succinyl oder Glutaryl.

Bevorzugt sind Verbindungen der Formel I, worin R Wasserstoff, die Acylgruppe einer Carbonsäure, eines Kohlensäurehalbesters, einer Carbaminsäure oder einer Thiocarbaminsäure mit bis zu 20 Kohlenstoffatomen, der Acylrest eines Polypeptids aus 2-200 der üblichen in der Natur vorkommenden Aminosäuren, der Rest einer Sulfonsäure mit bis zu 20 Kohlenstoffatomen, unsubstituiertes oder substituiertes Aminosulfonyl, Sulfo oder Phospho, und A C=O oder gegebenenfalls acyliertes C-OH bedeuten, worin C-OH durch eine Carbonsäure mit bis zu 20 Kohlenstoffatomen acyliert sein kann, wobei die gestrichelte Linie eine C=C-Doppelbindung an der Stelle der mittleren Bindung, wenn A C=O ist, oder an der Stelle der beiden äusseren Bindungen, wenn A gegebenenfalls acyliertes C-OH ist, symbolisiert, und Salze solcher Verbindungen.

Besonders bevorzugt sind Verbindungen der Formel I, worin R Wasserstoff, $C_1$-$C_{20}$-Alkanoyl, durch Hydroxy, verethertes oder verestertes Hydroxy, Amino, acyliertes Amino, Carboxy, amidiertes oder verestertes Carboxy, Oxo und/oder Halogen substituiertes $C_2$-$C_7$-Alkanoyl, Aroyl, $C_1$-$C_7$-Alkoxycarbonyl, $C_1$-$C_7$-Alkylaminocarbonyl, $C_1$-$C_7$-Alkylaminothiocarbonyl, Arylaminocarbonyl, Arylaminothiocarbonyl, der Acylrest eines Polypeptids aus 2-200 der üblichen in der Natur vorkommenden Aminosäuren, $C_1$-$C_7$-Alkansulfonyl, Arylsulfonyl, Aminosulfonyl, $C_1$-$C_7$-Alkylaminosulfonyl, Di($C_1$-$C_7$-alkyl)aminosulfonyl, Aryl-$C_1$-$C_4$-alkylaminosulfonyl, cyclisches fünf- oder sechsgliedriges Aminosulfonyl, worin der Cyclus ein bis zwei Stickstoffatome und gewünschtenfalls ein Sauerstoff- oder Schwefelatom enthält, Sulfo oder Phospho, und A C=O oder gegebenenfalls acyliertes C-OH, worin C-OH durch $C_1$-$C_{20}$-Alkanoyl, durch Hydroxy, verethertes oder verestertes Hydroxy, Amino, acyliertes Amino, Carboxy, amidiertes oder verestertes Carboxy, Oxo und/oder Halogen substituiertes $C_2$-$C_7$-Alkanoyl oder Aroyl acyliert sein kann, bedeuten, wobei die gestrichelte Linie die obengenannte Bedeutung hat, und Salze solcher Verbindungen.

Ganz besonders bevorzugt sind Verbindungen der Formel I, worin R Wasserstoff, einer der oben als bevorzugt genannten Acylreste oder Sulfonylreste, Sulfo oder Phospho, und A C=O oder gegebenenfalls acyliertes C-OH bedeuten, worin C-OH durch einen der oben als bevorzugt genannten Acylreste acyliert sein kann, wobei die gestrichelte Linie die obengenannte Bedeutung hat, und pharmazeutisch verwendbare Salze solcher Verbindungen.

In erster Linie betrifft die Erfindung Verbindungen der Formel I, worin R Wasserstoff, $C_1$-$C_7$-Alkanoyl, z.B. Formyl, Acetyl, 2,2-Dimethylpropionyl oder n-Hexanoyl, durch Carboxy, $C_1$-$C_4$-Alkoxycarbonyl oder Benzyloxycarbonyl und/oder Amino oder Benzyloxycarbonylamino substituiertes $C_2$-$C_7$-Alkanoyl, z.B. Succinyl oder Glutaryl mit einer freien Carboxygruppe, 3-Methoxycarbonylpropionyl, 4-Methoxycarbonylbutyryl, β-Aspartyl oder 3-Benzyloxycarbonyl-3-benzyloxycarbonylamino-propionyl, Phenylaminocarbonyl, Phenylaminothiocarbonyl, p-Toluolsulfonyl oder Sulfo, und A C=O oder gegebenenfalls acetyliertes C-OH bedeuten, wobei die gestrichelte Linie die obengenannte Bedeutung hat, und pharmazeutisch verwendbare Salze solcher Verbindungen.

Insbesondere betrifft die Erfindung die in den Beispielen genannten Verbindungen und deren pharmazeutisch verwendbaren Salze. Bevorzugte Verbindungen sind 21-Cyano-Saframycin Mx 1 und N-Formyl-21-cyano-Saframycin Mx 1.

Im folgenden werden die erfindungsgemässen Verbindungen als 21-Cyano-Saframycine Mx 1 bezeichnet. Ohne Zusatz bedeuten diese Bezeichnungen Verbindungen der Formel I, in denen A C-OH ist. Mit dem Zusatz "BC" (Bis-chinon) werden diese Bezeichnungen für Verbindungen verwendet, in denen A C=O bedeutet.

Erfindungsgemässe Salze von Verbindungen der Formel I sind in erster Linie pharmazeutisch verwendbare, nicht-toxische Säureadditionssalze. Beispiele für Säureadditionssalze mit nicht-toxischen, physiologisch gut verträglichen Säuren sind Salze mit anorganischen Säuren, z.B. Salzsäure, Schwefelsäure oder Phosphorsäure, oder mit organischen Carbon-, Sulfon-oder Sulfosäuren, z.B. Ameisensäure, Essigsäure, Propionsäure, Glykolsäure, Bernsteinsäure, Maleinsäure, Hydroxymaleinsäure, Methylmaleinsäure, Fumarsäure, Aepfelsäure, Weinsäure, Zitronensäure, Benzoesäure, Zimtsäure, Mandelsäure, Salicylsäure, 4-Aminosalicylsäure, 2-Phenoxybenzoesäure, 2-Acetoxybenzoesäure, Embonsäure, Nicotinsäure oder Isonicotinsäure, ferner Aminosäuren, z.B. α-Aminosäuren, sowie Methansulfonsäure, Ethansulfonsäure, 2-Hydroxyethansulfonsäure, Ethan-1,2-disulfonsäure, Benzolsulfonsäure, 4-Methylbenzolsulfonsäure oder Naphthalin-2-sulfonsäure, oder mit anderen sauren organischen Verbindungen, wie Ascorbinsäure.

Verbindungen, in denen R durch Carboxy substituiertes Alkanoyl oder Aroyl, Sulfo oder Phospho ist, können auch innere Salze oder Salze mit organischen Basen bilden. Bevorzugt sind Salze mit pharmazeutisch verwendbaren, nicht-toxischen Basen, beispielsweise mit physiologisch gut verträglichen organischen Aminen, z.B. mit tert-Butylamin, Hydroxyethylamin, Cyclohexylamin, Diethylamin, Di-n-Butylamin, Bis(hydroxyethyl)amin, Trimethylamin, Triethylamin, Tris(hydroxyethyl)amin oder Diethylhydroxyethylamin, ferner mit organischen Ammonium-Salzen, z.B. mit Tetramethylammonium oder Tetraethylammonium.

Zur Isolierung oder Reinigung können auch pharmazeutisch ungeeignete Salze Verwendung finden.

Die erfindungsgemässen Verbindungen der Formel I und ihre pharmazeutisch verwendbaren Salze weisen wertvolle pharmakologische Eigenschaften auf. Beispielsweise sind Verbindungen der Formel I in niedriger Konzentration von weniger als 0,3 µg/ml gegen verschiedene Keime wirksam, z.B. gegen Staphylococcus aureus, Bacillus subtilis, Micrococcus luteus und Escherichia coli. Neben antibiotischen Eigenschaften weisen die Verbindungen auch tumorhemmende Eigenschaften auf, beispielsweise gegen die experimentellen Tumoren Leukämie L 1210/S2, menschliches Melanom SK-MEL 109, menschliches Lungenkarzinom MBA 9812, Colon Adenokarzinom 26 und Reticulum Zellsarkom M5076.

Werden Mäuse mit Leukämie L1210/S2 beispielsweise mit 21-Cyano-Saframycin Mx 1, N-Caproyl-21-cyano-Saframycin Mx 1, N-Pivaloyl-21-cyano-Saframycin Mx 1 oder N-Formyl-21-Cyano-Saframycin Mx 1 an vier aufeinanderfolgenden Tagen mit intraperitonealen Injektionen von 1,25, 2,5 oder 5 mg/kg pro Tag behandelt, so verlängert sich ihre Lebensdauer signifikant um mehr als 70 %. Durch die Behandlung mit 21-Cyano-Saframycin Mx 1 (1,25 bis 5,0 mg/kg) werden 20 bis 60 % der Mäuse mit Leukämie L1210/S2 geheilt (Ueberlebenszeit mehr als 90 Tage). Das Gewicht von in Nacktmäuse implantiertem menschlichem Melanom SK-MEL 109 wird bei der Injektion von 1,25, 2,5 oder 5 mg/kg 21-Cyano-Saframycin Mx 1 pro Tag an sieben aufeinanderfolgenden Tagen im Vergleich zu demjenigen in unbehandelten Mäusen (gemessen nach 3 Wochen) um 83 %, 77 % beziehungsweise 92 % reduziert. Unter gleichen Bedingungen wird mit 1,25 oder 2,5 mg/kg N-Caproyl-21-cyano-Saframycin Mx 1 eine Reduktion um 35 % beziehungsweise 66 %, mit 2,5 und 5 mg/kg N-Pivaloyl-21-cyano-Saframycin Mx 1 um 46 % beziehungsweise 69 % und mit 2,5 und 5 mg/kg N-Formyl-21-cyano-Saframycin Mx 1 um 84 % beziehungsweise 73 % erreicht.

Verfahren

Verfahren zur Herstellung von Verbindungen der Formel I sind ebenfalls Gegenstand der Erfindung. Diese Verbindungen werden erhalten, indem man in einer Verbindung der Formel II

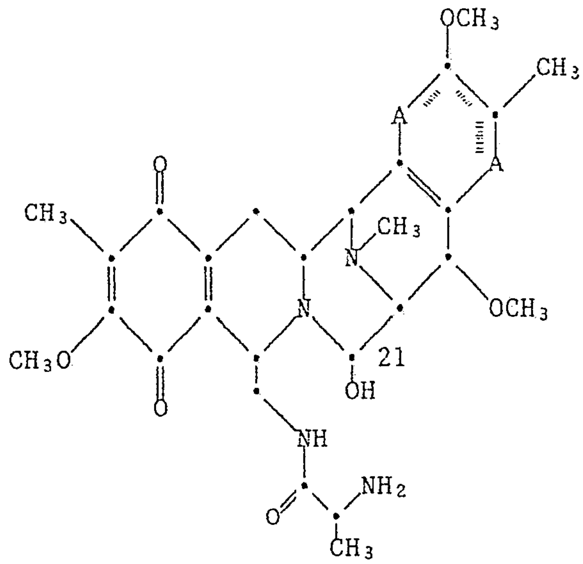

(II)

worin A C=O oder C-OH bedeuten, in beliebiger Reihenfolge die Hydroxygruppe am C-Atom 21 durch Cyanidierung in die Cyanogruppe und die $NH_2$-Gruppe durch Acylierung oder Sulfonierung in die NHR-Gruppe, worin R die unter Formel I angegebenen Bedeutungen hat, überführt, oder, zur Herstellung von Verbindungen der Formel I, worin R Waserstoff ist, in einer Verbindung der Formel II die Hydroxygruppe am C-Atom 21 durch Cyanidierung in die Cyanogruppe überführt, und, wenn erwünscht, eine erfindungsgemässe Verbindung in eine andere erfindungsgemässe Verbindung überführt und/oder ein erhältliches Salz in die freie Verbindung oder ein anderes Salz oder eine erhältliche freie Verbindung in ein Salz über-führt.

Unter Acylierung wird im folgenden auch die Einführung einer Sulfogruppe (Acyl = $-SO_3H$) oder einer Phosphogruppe (Acyl = $-PO_3H_2$) verstanden.

Die Cyanidierung erfolgt durch Umsetzung mit einer wässrigen Lösung eines Cyanidsalzes. Als Cyanidsalze kommen in erster Linie Alkalimetallcyanide, z.B. Natriumcyanid oder Kaliumcyanid, in Betracht, ferner auch Cyanidsalze von zweibasischen Metallen, z.B. Zinkcyanid, und Ammoniumcyanide, z.B. unsubstituiertes Ammoniumcyanid, Trialkylammoniumcyanide, z.B. Triethylammoniumcyanid, Tetraalkylammoniumcyanide, z.B. Tetramethyl- oder Tetrabutylammoniumcyanid, oder Benzyltrialkylammoniumcyanide, z.B. Benzyltrimethylammoniumcyanid. Die Umsetzung erfolgt in wässriger oder wässrig-organischer Lösung, vorzugsweise in Pufferlösungen im schwach sauren Bereich oder nahe dem Neutralpunkt, z.B. bei einem pH zwischen 5 und 8, un bei Temperaturen zwischen 0°C und etwa 40°C, vorzugsweise um Raumtemperatur. Die Reaktionsdauer beträgt wenige Minuten bis einige Stunden, beispielsweise 10 bis 30 Minuten, abhängig von der Temperatur und dem pH des Reaktionsgemisches. Vorzugsweise erfolgt die Umsetzung in einer inerten Gas-Atmosphäre, z.B. unter Stickstoff. Wird Luftsauerstoff nicht völlig ausgeschlossen, so wird unter den Reaktionsbedingungen eine Verbindung der Formel I, worin A C-OH bedeutet, zum entsprechenden Chinon mit der Bedeutung A C=O aufoxidiert.

Die Acylierung oder Sulfonylierung einer Verbindung der Formel II, oder einer durch Cyanidierung erhältlichen Verbindung der Formel I, worin R Wasserstoff bedeutet, erfolgt nach an sich bekannten Methoden, beispielsweise durch Umsatz mit einer Säure der Formel R-OH oder einem reaktiven funktionellen Derivat davon.

Wenn eine freie Carbonsäure für die Acylierung verwendet wird, wird die Reaktion üblicherweise in Gegenwart eines geeigneten Kondensationsmittels durchgeführt, z.B. eines Carbodiimids, beispielsweise Dicyclohexyl- oder vorzugsweise N-Ethyl-N'-3-dimethylaminopropyl-carbodiimid. Die Kondensation erfolgt in einem gepufferten wässrig-organischen Lösungsmittelgemisch oder einem wässrigen Puffer nahe dem Neutralpunkt, z.B. zwischen pH 6 und 8, gegebenenfalls unter Kühlung oder Erwärmung und in einer inerten Gas-Atmosphäre, z.B. unter Stickstoff.

Ein reaktives funktionelles Derivat einer Carbonsäure, eines Kohlensäurehalbesters oder einer Sulfonsäure ist ein entsprechendes Anhydrid, beispielsweise ein symmetrisches anhydrid, z.B. Essigsäureanhydrid, ein gemischtes Anhydrid der Säure $R^2$-OH mit einer anorganischen oder organischen Säuren, z.B. mit Halogenwasserstoffsäure, wie Salzsäure oder Bromwasserstoffsäure, also beispielsweise ein Carbonsäurechlorid oder -bromid, ein Chlorameisensäureester oder ein Sulfonsäurechlorid, oder ein gemischtes Anhydrid einer Carbonsäure mit einer anderen Carbonsäure, also z.B. Ameisensäure-Essigsäure-Anhydrid, oder mit einem Halbester der Kohlensäure, z.B. dem Ethyl- oder Isobutyl-halbester der Kohlensäure. Ein weiteres reaktives funktionelles Derivat einer Carbonsäure oder eines Kohlensäurehalbesters ist beispielsweise ein Aktivierter Ester, z.B. ein N-Hydroxyester, wie der Ester mit N-Hydroxypiperidin, N-Hydroxysuccinimid,

N-Hydroxyphthalimid oder 1-Hydroxybenztriazol. Ein reaktives funktionelles Derivat einer Carbaminsäure oder einer Thiocarbaminsäure ist das entsprechende Isocyanat beziehungsweise Isothiocyanat. Zur Einführung einer gegebenenfalls substituierten Aminosulfonylgruppe wird das entsprechend substituierte Sulfamidsäurechlorid, beispielsweise unsubstituiertes Sulfamidsäurechlorid, Dimethylsulfamidsäurechlorid, tert-Butylsulfamidsäurechlorid oder Sulfopyrrolidinsäurechlorid verwendet. Anstelle des Chlorids kann auch ein aktivierter Ester eingesetzt werden, beispielsweise ein Ester eines Phenols mit elektronenziehenden Substituenten, z.B. mit Pentafluorophenol oder mit 2,4,5-Trichlorphenol.

Zur Einführung der Sulfogruppe verwendet man Chlorsulfonsäure oder vorzugsweise einen Komplex von Schwefeltrioxid mit einem organischen Amin, beispielsweise mit Trimethylamin, Triethylamin, Benzyldimethylanilin, N-Methylmorpholin oder Pyridin. Zur Einführung der Phosphorgruppe eignet sich ein reaktives Amid der Phosphorsäure, beispielsweise Phosphorsäureimidazolid. Geeignet sind auch Ester, beispielsweise Ester mit Salzsäure und gemischte Ester mit Salzsäure und einem leicht abspaltbaren Alkohol, beispielsweise Phosphoroxychlorid, Phosphorsäure-bis(2,2,2- trichlorethylester)-chlorid, Phosphorsäure-2,2,2-tribromethylester-chlorid-morpholid, Phosphorsäure-2,2,2-tribromethylester-dichlorid und Phosphorsäure-2,2,2-trichlorethylester-dichlorid. Verwendet man einen Salzsäureester oder einen entsprechenden gemischten Ester, so schliesst sich an die Acylierung noch eine Esterhydrolyse beziehungsweise Esterspaltung nach an sich bekannten Methoden an, um die gewünschte Phospho-Gruppe freizusetzen.

Die Acylierungs- oder Sulfonylierungsreaktionen mit reaktiven funktionellen Derivaten von R-OH werden in organischer Lösung oder vorzugsweise in wässrig-organischen homogenen oder zweiphasigen Lösungsmittelgemischen durchgeführt, wenn erwünscht unter Kühlung oder leichter Erwärmung, z.B. im Temperaturbereich von ungefähr -30° C bis ungefähr 50° C, vorzugsweise um Raumtemperatur, und gegebenenfalls in einer Inertgas-Atmosphäre, z.B. unter Stickstoff. Dabei wird ein Säureakzeptor zugegeben, beispielsweise eine geeignete organische Base, z.B. ein Amin, z.B. Trimethylamin, Triethylamin, N-Methylmorpholin, 1,5-Diazabicyclo[4.3.0]non-5-en, 1,8-Diazabicyclo[5.4.0]undec-7-en, Pyridin oder Dimethylaminopyridin, oder eine anorganische Base, beispielsweise ein Alaklimetall- oder Erdalkalimetall-hydroxyd, z.B. Natrium-, Kalium- oder Calcium-hydroxid, ein Alkalimetall- oder Erdalkalimetall-carbonat, z.B. Natriumcarbonat, Natriumhydrogencarbonat oder Calciumcarbonat, oder ein Alkalimetallphosphat, z.B. Natrium- oder Kalium-phosphat oder -hydrogenphosphat. Vorzugsweise wird die Acylierungsreaktion in einem wässrigen Puffer nahe dem Neutralpunkt, z.B. zwischen pH 6 und pH 8, durchgeführt, wobei die Pufferbase die Rolle des Säureakzeptors übernimmt. Gewünschtenfalls wird der pH durch kontinuierliche Zugabe einer anorganischen Base, z.B. Natriumhydroxid, im Bereich von 6 bis 8 gehalten. Als Lösungsvermittler wird vorzugsweise ein mit Wasser mischbares organisches Lösungsmittel eingesetzt, beispielsweise Tetrahydrofuran, Dioxan oder Acetonitril. Arbeitet man in reinem organischem Lösungsmittel, so verwendet man vorzugsweise Pyridin.

Um die Acylierung einer phenolischen Hydroxygruppe A zurückzudrängen, wird dem wässrig-organischen Lösungsmittelgemisch beispielsweise ein Alkohol, z.B. Methanol oder Ethanol, zugegeben. Ist jedoch die Acylierung der phenolischen Hydroxygruppe A erwünscht, so wird mit einem ent sprechend grossen Ueberschuss an Acylierungsmittel gearbeitet.

Die Umwandlung einer Verbindung der Formel I in eine andere Verbindung der Formel I geschieht mit an sich bekannten Methoden, beispielsweise durch Oxidation, durch Reduktion, durch Acylierung, Sulfonylierung oder durch an sich bekannte Umwandlung einer Acylgruppe.

Umwandlungen von Hydrochinonen, d.h. Verbindungen der Formel I, worin A C-OH bedeutet, in entsprechende Chinone, d.h. Verbindungen der Formel I, worin A C=O bedeutet, durch Oxidation und Umwandlungen der Chinone in Hydrochinone durch Reduktion können beispielsweise analog den in "Methoden der Organischen Chemie (Houben-Weyl)", 4. Auflage, Band VII/3a, Thieme Verlag Stuttgart 1977, beschriebenen Verfahren durchgeführt werden. Geeignete milde Oxidationsmittel sind beispielsweise Metallsalze und Metalloxide, beispielsweise Silber(I)-oxid in Diethylether, Benzol oder Toluol in Gegenwart eines Trockenmittels, z.B. Natriumsulfat, Eisen(III)salze, wie Eisen(III)chlorid in Wasser oder wässrigem Ethanol, Kupfersalze oder Thalliumsalze, z.B. Thallium(III)trifluoracetat. Ebenfalls geeignet als Oxidationsmittel ist Luftsauerstoff in neutraler Lösung, z.B. in Pufferlösungen pH 7 bis pH 8. Geeignete Reduktionsmittel sind beispielsweise Wasserstoff in Gegenwart heterogener Katalysatoren, z.B. Platinoxid oder Palladium auf Kohle, oder homogener Katalysatoren, z.B. Tris(triphenylphosphin)rhodium(I)chlorid, ferner Metallhydride, z.B. Borhydride, wie Natriumborhydrid, oder Aluminiumhydride, wie Lithiumaluminiumhydrid, reduzierende Salze, z.B. Natriumdithionit, ferner auch Licht in Gegenwart eines Wasserstoff-Donators, beispielsweise gewöhnliches Tageslicht und Methanol. Umwandlungen der Chinone und Hydrochinone ineinander können auch durch elektrochemische Methoden, beispielsweise Elektrolyse in schwach sauren, salzhaltigen Lösungen, z.B. Pufferlösungen pH 4 bis pH 6, vorgenommen werden.

Acylierungen oder Sulfonylierungen erfolgen in der oben dargestellten Art und Weise. Soll eine Verbindung der Formel I, worin R Acyl und A C-OH bedeuten, an den phenolischen Gruppen acyliert werden, so kann anstelle des üblichen wässrigen oder wässrig-organischen Lösungsmittels auch ein rein organisches Lösungsmittel in Gegenwart einer organischen Base eingesetzt werden. Als Lösungsmittel sind beispielsweise Ether, z.B. Tetrahydrofuran oder Dimethoxyethan, Amide, z.B. Diemthylformamid oder Dimethylacetamid, oder Acetonitril, jeweils in Gegenwart einer der obengenannten Amin-Basen, geeignet. Die Reaktion kann auch direkt im Pyridin durchgeführt werden.

Umwandlungen von Acylgruppen R erfolgen in an sich bekannter Weise. Dabei sind die Reaktionsbedingungen jedoch so zu wählen, dass die übrigen funktionellen Gruppen der erfindungsgemässen Verbindungen

nicht verändert werden. Es ist beispielsweise möglich, in einer Acylgruppe R, in der eine Aminogruppe durch Benzyloxycarbonyl und/oder eine Carboxygruppe durch Benzyl geschützt sind, die genannten Gruppen durch milde Hydrogenolyse freizusetzen, beispielsweise mit Wasserstoff in Gegenwart eines Edelmetallkatalysators, z.B. Palladium auf Kohle.

Wenn Verbindungen der Formel I, worin R Acyl, Sulfonyl, Sulfo oder Phospho bedeuten, hergestellt werden sollen, kann das Ausgangsmaterial der Formel I entweder zuerst cyanidiert und anschliessend acyliert oder sulfonyliert, oder zuerst acyliert oder sulfonyliert und dann cyanidiert werden. Die entstehenden Zwischenprodukte können nach Durchführung der ersten Stufe entweder isoliert oder ohne Isolierung der zweiten Stufe unterworfen werden.

Salze von Verbindungen der Formel I können in an sich bekannter Weise hergestellt werden, beispielsweise durch Umsetzen der freien Verbindung mit vorzugsweise stöchiometrischen Mengen oder einem kleinen Ueberschuss der ein Säureadditionssalz bildenden Säure oder des salzbildenden organischen Amins.

Säureadditionssalze können in üblicher Weise in die freien Verbindungen oder in Salze mit Basen übergeführt werden, beispielsweise durch Behandeln mit einer äquimolaren Menge einer freien Base, z.B. eines Hydroxids, wie Alkalihydroxids, z.B. Lithium-, Kalium- oder Natriumhydroxids, Erdalkalihydroxids, z.B. Calciumhydroxids oder Magnesiumhydroxids, oder Ammoniumhydroxids, z.B. unsubstituierten Ammoniumhydroxids oder Benzyltrimethylammoniumhydroxids, oder eines organischen tertiären Amins, z.B. Triethylamin. Dabei ist jedoch zu beachten, dass die freie Verbindung der Formel I nur beschränkt haltbar ist und zur Lagerung in ein Säure additionssalz übergeführt werden muss.

Salze mit organischen Aminen werden gleichfalls in üblicher Weise in die freien Verbindungen oder in Säureadditionssalze übergeführt, beispielsweise durch Behandeln mit einer äquimolaren Menge einer Mineralsäure, z.B. Salzsäure, Schwefelsäure oder Phosphorsäure, oder einer organischen starken Säure, beispielsweise Ameisensäure, Trifluoressigsäure oder p-Toluolsulfonsäure.

Salze von Verbindungen der Formel I werden in an sich bekannter Weise in andere Salze überführt. Vorzugsweise geschieht die Umwandlung von Salzen in andere Salze mit Ionenaustauschern, die mit dem gewünschten Anion oder Kation beladen sind, oder durch Adsorptionschromatographie in einem Lösungsmittel, das die Säure des gewünschten Säureadditionssalzes oder das gewünschte organische Amin oder Ammoniumion im Ueberschuss enthält.

Die Erfindung betrifft auch diejenigen Ausführungsformen des Verfahrens, bei der man von einem auf irgendeiner Stufe des Verfahrens erhältlichen Zwischenprodukt ausgeht und die abschliessenden Schritte durchführt, das Verfahren auf irgendeiner Stufe abbricht und/oder eine nach dem erfindungsgemässen Verfahren erhältliche Verbindung in situ weiterverarbeitet.

Ausgangsmaterialien und Zwischenprodukte

Verbindungen der Formel II, worin A C=O oder C-OH Bedeuten, sind Erfindungsgegenstand der Europäischen Patentanmeldung Nr. 87810454.6. Sie werden beispielsweise hergestellt, indem man den Stamm Mx x48 der Species Myxococcus xanthus in einer Kultur enthaltend Kohlenstoff- und Stickstoffverbindungen und essentielle anorganische Salze in leicht assimilierbarer Form bei einer Temperatur zwischen 15°C und 40°C und einem pH-Wert zwischen 5 und 9 unter aeroben Bedingungen züchtet und die gebildeten Verbindungen der Formel I isoliert.

Der Mikroorganismus Myxococcus xanthus, Stamm Mx x48, ist bei der National Collection of Industrial and Marine Bacteria, Aberdeen, Schottland, unter der Nummer NCIB 12 268 hinterlegt.

Die zur Züchtung verwendbare Kultur muss eine Kohlenstoff- und Stickstoffquelle sowie essentielle anorganische Salze enthalten. Als Kohlenstoffquelle und Stickstoffquelle geeignet sind Aminosäuren, Peptide und Proteine sowie deren Abbauprodukte wie Pepton oder Trypton, ferner Fleischextrakte, Getreidemehl, z.B. von Mais oder Weizen, Bohnen, insbesondere Sojabohnen, Fischmehl, Samen, beispielsweise der Baumwollpflanze, Destillationsrückstände der Alkoholherstellung, Hefeextrakte usw. Als essentielle anorganische Salze kann die Nährlösung beispielsweise Chloride, Carbonate, Sulfate, Phosphate von Alkali- oder Erdalkalimetallen, z.B. Natrium, Kalium, Magnesium und Calcium, ferner Salze von Eisen, Zink, Mangan, Molybdän und Kupfer enthalten. Die Züchtung erfolgt bevorzugt in Flüssigkulturen, insbesondere wässrigen Kulturen.

Als flüssiges Kulturmedium eignet sich insbesondere das Medium MDI (Pepton aus Casein, tryptisch verdaut, 0,3 %; $CaCl_2 \cdot 2H_2O$ 0,05 %; $MgSO_4 \cdot 7H_2O$ 0,2 %). Andere geeignete flüssige Kulturmedien bestehen beispielsweise aus Pepton aus Casein 0,05 %, $CaCl_2 \cdot 2H_2O$ 0,05 %, $MgSO_4 \cdot 7H_2O$ 0,2 %, dem wahlweise 0,5 % Einzellerprotein, Maisquellwasser, Hefeextrakt, Aminosäurehydrolysat aus Casein oder Protein aus Fischmehl zugegeben wird.

Die Züchtung erfolgt bevorzugt bei Temperaturen zwischen 25°C und 35°C, z.B. um 30°C. Geeignete pH-Werte liegen zwischen pH 6,5 und pH 8,5.

Vorzugsweise züchtet man in mehreren Stufen, indem man eine Vorkultur, z.B. in einem der genannten Kulturmedien, herstellt (Inoculum), welche man anschliessend nach ca. ein- bis zweitägiger Fermentation in eine grössere Kultur in einem Verdünnungsverhältnis zwischen 1:10 und 1:500 überimpft. Diese Kultur kann wiederum nach ca. zwei- bis dreitägiger Fermentation in eine noch grössere Hauptkultur in einem Verdünnungsverhältnis von 1:10 bis 1:100 überimpft werden.

Der Fermentationsverlauf wird durch Probenentnahme analytisch während der Fermentation verfolgt, z.B. durch Messung des pH-Wertes der Kultur, welcher während der Fermentation von ca. pH 7,2 auf etwa pH 8,0

ansteigt, der optischen Dichte, welche ein Mass für das Wachstum des Stammes ist, gravimetrisch anhand des Trockengewichts der gebildeten Zellmasse, durch Dünnschichtchromatographie, durch Hochdruck-Flüssigchromatographie an Umkehrphasen oder durch Bestimmung der antibiotischen Aktivität der im Kulturfiltrat enthaltenen Komponenten.

Für die Isolierung der gewünschten Verbindungen aus der rohen Fermentationsbrühe wird diese mehrere Stunden mit makroporösen, nicht-ionischen Absorberharzen gerührt. Geeignete Adsorberharze haben ein Porenvolumen von ca. 0,5 bis ca. 4,5 ml/g, eine spezifische Oberfläche von ca. 100-1000 $m^2/g$ und einen durchschnittlichen Porendurchmesser von ca. 4 bis ca. 130 nm und sind beispielsweise unter den Handelsnamen AMBERLITE® XAD ER-180 der Fa. Röhm & Haas erhältlich.

Nach Abtrennung der Fermentationsbrühe vom Adsorberharz wird dieses mit Wasser gewaschen und anschliessend mit Isopropanol eluiert. Die Eluate werden angesäuert und im Vakuum eingeengt.

Die rohen Extrakte werden mit chromatographischen Methoden gereinigt, beispielsweise durch Chromatographie an einem Ionenaustauscher mit Carboxy-Gruppen, Adsorptions- bzw. Verteilungschromatographie und/oder Hochdruck-Flüssigchromatographie an Silicagel enthaltend langkettige Alkylgruppen ("reversed phase").

Zwischenprodukte der Formel

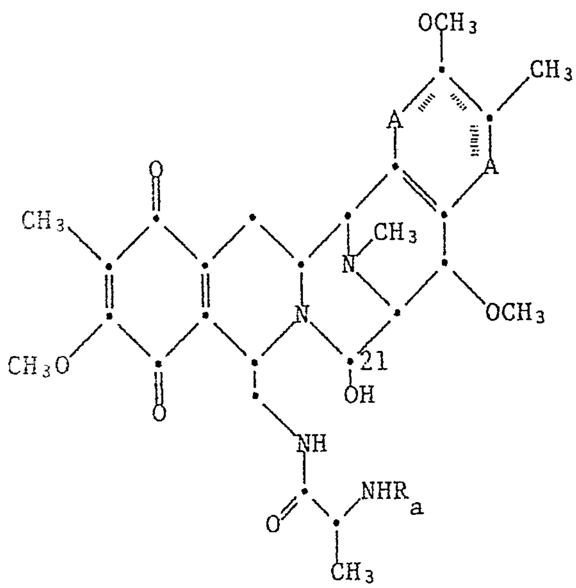

(IIa),

worin $R_a$ Acyl ist und A C=O oder gegebenenfalls acyliertes C-OH bedeutet, sind ebenfalls Erfindungsgegenstand der Europäischen Patentanmeldung Nr. 87810454.6. Sie können aus einer Verbindung der Formel II nach einem der vorstehenden Acylierungsverfahren erhalten werden.

Zwischenprodukte der Formel

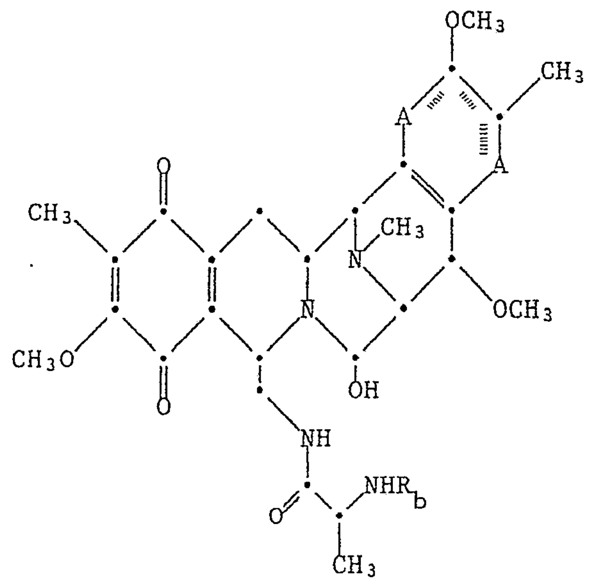

(IIb),

worin $R_b$ durch Carboxy oder verestertes Carboxy substituiertes Acyl, Sulfo oder Phospho und A C=O oder gegebenenfalls acyliertes C-OH bedeuten, wobei die gestrichelte Linie eine C=C-Doppelbindung an der Stelle der mittleren Bindung, wenn A C=O ist, oder an der Stelle der beiden äusseren Bindungen, wenn A gegebenenfalls acyliertes C-OH ist, symbolisiert, und Salze solcher Verbindungen, insbesondere pharmazeutisch verwendbare Salze, sind neu und ebenfalls Gegenstand der vorliegenden Erfindung.

Für die Konfigurationen an den chiralen Zentren der Verbindungen der Formel II, IIa und IIb gilt das gleiche wie für diejenigen der Formel I.

In den Verbindungen Der Formel IIb haben Acyl, Sulfo, Phospho und A die gleichen Bedeutungen, wie unter der Formel I angegeben.

Acyl ist beispielsweise substituiertes $C_1$-$C_{20}$-Alkanoyl, insbesondere $C_1$-$C_7$-Alkanoyl, $C_1$-$C_7$-Alkenoyl, Aroyl mit bis zu 20 C-Atomen oder Heteroaroyl mit bis zu 20 C-Atomen und 1 bis 2 Heteroatomen, beispielsweise Stickstoff, Sauerstoff und/oder Schwefel. Die genannten Acylreste können ausser den einfach oder mehrfach vorkommenden Carboxy-und/oder veresterten Carboxyresten auch noch andere Substituenten tragen, beispielsweise Amino, Acylamino, z.B. $C_1$-$C_7$-Alkanoylamino, $C_1$-$C_7$-Alkoxycarbonylamino oder Aryl-$C_1$-$C_4$-alkoxycarbonylamino, Hydroxy, $C_1$-$C_7$-Alkoxy und/oder $C_1$-$C_7$-Alkanoyloxy. Die genannten Amino- oder Hydroxysubstituenten können auch durch eine in der Peptid- oder Alkaloidchemie übliche Schutzgruppe geschützt sein.

Der Substituent verestertes Carboxy ist beispielsweise $C_1$-$C_7$-Alkoxycarbonyl oder Aryl$C_1$-$C_4$-alkoxycarbonyl.

Sulfo bedeutet den Schwefelsäurerest $-SO_3H$. Phospho bedeutet den Phosphorsäurerest $-PO_3H_2$.

In der Gruppe A mit der Bedeutung acyliertes C-OH ist die Acylgruppe beispielsweise eine der obengenannten Acylgruppen, insbesondere $C_1$-$C_7$-Alkanoyl, durch Carboxy substituiertes $C_1$-$C_7$-Alkanoyl oder durch $C_1$-$C_7$-Alkoxycarbonyl oder Aryl$C_1$-$C_4$-alkoxycarbonyl substituiertes $C_1$-$C_7$-Alkanoyl.

$C_1$-$C_{20}$-Alkanoyl ist vorzugsweise $C_1$-$C_7$-Alkanoyl, beispielsweise Formyl, Acetyl, Propionyl, n-Butyryl, 2-Methylpropionyl, n-Pentanoyl, 2,2-Dimethylpropionyl, 2-Methylbutyryl, 3-Methylbutyryl oder n-Hexanoyl, oder geradkettiges $C_8$-$C_{20}$-Alkanoyl mit einer geraden Anzahl Kohlenstoffatome, beispielsweise n-Octanoyl, n-Decanoyl, n-Dodecanoyl, n-Tetradecanoyl, n-Hexadecanoyl, n-Octadecanoyl oder n-Icosanoyl.

$C_1$-$C_7$-Alkenoyl ist beispielsweise Acryloyl, Crotonoyl oder Allylcarbonyl.

Aroyl ist beispielsweise Benzoyl, 1- oder 2-Naphthoyl, Indan-1- oder 2-carbonyl, Inden-1, 2- oder 5-carbonyl, 4-Biphenylylcarbonyl oder 9-Fluorenylcarbonyl.

Heteroaroyl ist beispielsweise 2- oder 3-Furoyl, 2- oder 3-Thenoyl, Pyrrol-2- oder 3-carbonyl, Pyridin-2-, 3- oder 4-carbonyl, Indol-3-oder 5-carbonyl, Chinolin-2-carbonyl oder Isochinolin-1-carbonyl.

$C_1$-$C_7$-Alkoxy ist beispielsweise Methoxy, Ethoxy, Propoxy, Isopropoxy, n-Butoxy, tert-Butoxy, tert-Pentoxy oder n-Hexoxy. Aryl-$C_1$-$C_4$-alkoxy ist beispielsweise unsubstituiertes oder substituiertes Benzyloxy, z.B. p-Nitrobenzyloxy, 2-Phenyl-2-propoxy oder 2-Phenylethoxy.

Beispiele für Reste Acyl $R_b$ der erfindungsgemässen Verbindungen der Formel IIb sind Carboxy-$C_1$-$C_7$-alkanoyl, z.B. Oxalyl, Malonyl, Succinyl, Glutaryl oder Adipoyl mit einer freien Carboxygruppe oder entsprechend veresterte Reste, z.B. Methoxalyl, Ethoxalyl, Methoxy-, Ethoxy- oder Benzyloxycarbonylacetyl, Methoxy-, Ethoxy- oder Benzyloxycarbonyl-3-propionyl, Methoxy- oder Ethoxycarbonyl-4-butyryl oder Methoxy-oder Ethoxycarbonyl-5-pentanoyl, Carboxy-$C_1$-$C_4$-alkenoyl, z.B. Fumaroyl, Maleoyl, Citraconoyl oder Mesaconoyl mit einer freien Carboxygruppe oder entsprechend veresterte Reste, z.B. cis- oder trans-Methoxy- oder

Ethoxycarbonyl-β-acryloyl, Carboxy-aroyl, z.B. Phthaloyl, Isophthaloyl oder Terephthaloyl mit einer freien Carboxygruppe, 3-Carboxy-2-naphthoyl oder 4′-Carboxybiphenylyl-4-carbonyl, oder entsprechend veresterte Reste, z.B. 2-, 3- oder 4-Methoxy- oder Ethoxycarbonylbenzoyl, 3-Methoxy- oder Ethoxycarbonyl-2-naphthoyl oder 4′-Methoxy- oder Ethoxycarbonyl-biphenyl-yl-4-carbonyl, Carboxy-heteroaroyl, z.B. 3-Carboxy-4-furoyl, 3-Carboxy-4-thenoyl, 2-Carboxynicotinoyl oder 2- oder 3-Carboxyisonicotinoyl, oder entsprechend veresterte Reste, z.B. 3-Methoxy- oder Ethoxycarbonyl-4-furoyl oder 3-Methoxy- oder Ethoxycarbonyl-4-thenoyl, Aminocarboxy-$C_1$-$C_7$-alkanoyl, z.B. α- oder β-Aspartyl oder α- oder γ-Glutamyl, oder entsprechend veresterte und/oder acylierte Reste, z.B. 3-Benzyloxycarbonyl-2- oder 3-benzyloxycarbonylamino-propionyl, 3-tert-Butoxycarbonyl-2- oder 3-tert-butoxycarbonylamino-propionyl oder 4-Benzyloxycarbonyl-2- oder 4-benzyloxycarbonylamino-butyryl, Hydroxy-carboxy-$C_1$-$C_7$-alkanoyl, z.B. Maloyl oder Tartaroyl mit einer freien Carboxygruppe oder entsprechende an der Carboxygruppe veresterte und/oder an der Hydroxygruppe veresterte oder veretherte Reste, z.B. 3-Methoxy carbonyl-2-hydroxy-, methoxy- oder acetoxy-propionyl oder 3-Methoxycarbonyl-2,3-dihydroxy-, dimethoxy- oder diacetoxy-propionyl, Sulfo oder Phospho.

Beispiele für Acylreste in der Gruppe A mit der Bedeutung acyliertes C-OH sind Acetyl, Propionyl, Pivaloyl oder n-Hexanoyl, ferner 3-Carboxypropionyl, 3-Methoxy- oder Ethoxycarbonylpropionyl, 2-Carboxybenzoyl oder 2-Methoxy- oder Ethoxycarbonylbenzoyl.

Bevorzugt sind Verbindungen der Formel $II_b$, worin $R_b$ Carboxy-$C_1$-$C_7$-alkanoyl, z.B. Oxalyl, Malonyl, Succinyl, Glutaryl oder Adipoyl mit einer freien Carboxygruppe, $C_1$-$C_7$-Alkoxycarbonyl-$C_1$-$C_7$-alkanoyl, z.B. Methoxalyl, Ethoxalyl, Methoxy- oder Ethoxycarbonylacetyl, 3-Methoxy-, Ethoxy- oder tert-Butoxycarbonylpropionyl oder 4-Methoxy- oder Ethoxycarbonylbutyryl, Aryl-$C_1$-$C_4$-alkoxycarbonyl-$C_1$-$C_7$-alkanoyl, z.B. Benzyloxycarbonylacetyl, 3-Benzyloxycarbonylpropionyl oder 4-Benzyloxycarbonylbutyryl, Carboxy-aroyl, z.B. 2-, 3- oder 4-Carboxybenzoyl oder 4′-Carboxybiphenylyl-4-carbonyl, $C_1$-$C_7$-Alkoxycarbonyl-aroyl, z.B. 2-, 3-oder 4-Methoxycarbonylbenzoyl, 2-, 3- oder 4-Ethoxycarbonylbenzoyl oder 4′-Methoxy- oder Ethoxycarbonyl-biphenylyl-4-carbonyl, Amino-carboxy-$C_1$-$C_7$-alkanoyl, z.B. α- oder β-Aspartyl oder α- oder γ-Glutamyl, Acylamino-$C_1$-$C_7$-alkoxycarbonyl-$C_1$-$C_7$-alkanoyl, z.B. 3-tert-Butoxycarbonyl-2- oder 3-tert-butoxycarbonylamino-propionyl, Acylamino-aryl-$C_1$-$C_4$-alkoxycarbonyl-$C_1$-$C_7$-alkanoyl, z.B. 3-Benzyloxycarbonyl-2- oder 3-benzyloxycarbonylamino-propionyl, Hydroxy-carboxy-$C_1$-$C_7$-alkanoyl, z.B. 3-Carboxy-2-hydroxy- oder 2,3-dihydroxy-propionyl, Acyloxy-$C_1$-$C_7$-alkoxycarbonyl-$C_1$-$C_7$-alkanoyl, z.B. 3-Methoxycarbonyl-2-acetoxy- oder 2,3-diacetoxy-propionyl oder 3-Ethoxycarbonyl-2-acetoxy- oder 2,3-diacetoxy-propionyl, Sulfo oder Phospho, und A C=O oder gegebenfalls acyliertes C-OH bedeuten, worin C-OH durch $C_1$-$C_7$-Alkanoyl, z.B. Acetyl, Propionyl, Pivaloyl oder n-Hexanoyl, Carboxy-$C_1$-$C_7$-alkanoyl, z.B. 3-Carboxy-propionyl, $C_1$-$C_7$-Alkoxycarbonyl-$C_1$-$C_7$-alkanoyl, z.B. 3-Methoxy-oder Ethoxycarbonyl-propionyl, Carboxy-aroyl, z.B. 2-Carboxybenzoyl, oder $C_1$-$C_7$-Alkoxycarbonyl-aroyl, z.B. 2-Methoxy- oder Ethoxycarbonylbenzoyl, substituiert sein kann, wobei die gestrichelte Linie eine C=C-Doppelbindung an der Stelle der mittleren Bindung, wenn A C=O ist, oder an der Stelle der beiden äusseren Bindungen, wenn A gegebenenfalls acyliertes C-OH ist, symbolisiert, und Salze solcher Verbindungen.

Besonders bevorzugt sind Verbindungen der Formel $II_b$, worin $R_b$ Carboxy-$C_1$-$C_7$-alkanoyl, z.B. Malonyl, Succinyl oder Glutaryl mit einer freien Carboxygruppe, $C_1$-$C_7$-Alkoxycarbonyl-$C_1$-$C_7$-alkanoyl, z.B. Methoxy- oder Ethoxycarbonylacetyl, 3-Methoxy- oder Ethoxycarbonylpropionyl oder 4-Methoxy- oder Ethoxycarbonylbutyryl, Carboxy-aroyl, z.B. 2-, 3- oder 4-Carboxybenzoyl, $C_1$-$C_7$-Alkoxycarbonyl-aroyl, z.B. 2-, 3- oder 4-Methoxycarbonylbenzoyl oder 2-, 3- oder 4-Ethoxycarbonylbenzoyl, Aminocarboxy-$C_1$-$C_7$-alkanoyl, z.B. α- oder β-Aspartyl oder α- oder γ-Glutamyl, Sulfo oder Phospho, und A C=O oder gegebenenfalls acyliertes C-OH bedeuten, worin C-OH durch $C_1$-$C_7$-Alkanoyl, z.B. Acetyl, Propionyl oder Pivaloyl, Carboxy-$C_1$-$C_7$-alkanoyl, z.B. 3-Carboxypropionyl, oder $C_1$-$C_7$-Alkoxycarbonyl-$C_1$-$C_7$alkanoyl, z.B. 3-Methoxy- oder Ethoxycarbonylpropionyl, substituiert sein kann, wobei die gestrichelte Linie die obengenannte Bedeutung hat, und Salze dieser Verbindungen.

In erster Linie betrifft die Erfindung Verbindungen der Formel $II_b$, worin $R_b$ Carboxy-$C_1$-$C_7$-alkanoyl, z.B. Succinyl oder Glutaryl mit einer freien Carboxygruppe, $C_1$-$C_7$-Alkoxycarbonyl-$C_1$-$C_7$-alkanoyl, z.B. 3-Methoxycarbonylpropionyl oder 4-Methoxycarbonylbutyryl, Carboxybenzoyl, z.B. 2-, 3- oder 4-Carboxybenzoyl, oder Sulfo, und A C=O oder gegebenfalls acetyliertes C-OH bedeuten, wobei die gestrichelte Linie die obengenannte Bedeutung hat, und pharmazeutisch verwendbare Salze solcher Verbindungen.

Insbesondere betrifft die Erfindung die in den Beispielen genannten Verbindungen und deren pharmazeutisch verwendbaren Salze.

Im folgenden werden die erfindungsgemässen Verbindungen der Formel $II_b$ als N-Acyl-Saframycine Mx 1 bezeichnet. Ohne Zusatz bedeuten diese Bezeichnungen Verbindungen der Formel I, in denen A C-OH ist. Mit dem Zusatz "BC" (Bis-chinon) werden diese Bezeichnungen für Verbindungen verwendet, in denen A C=O bedeutet.

Erfindungsgemässe Salze von Verbindungen der Formel $II_b$ sind beispielsweise pharmazeutisch verwendbare, nicht-toxische Säureadditionssalze. Beispiele für Säureadditionssalze mit nicht-toxischen, physiologisch gut verträglichen Säuren sind Salze mit anorganischen Säuren, z.B. Salzsäure, Schwefelsäure oder Phosphorsäure, oder mit organischen Carbon-, Sulfon-oder Sulfonsäuren, z.B. Ameisensäure, Essigsäure, Propionsäure, Glykolsäure, Bernsteinsäure, Maleinsäure, Hydroxymaleinsäure, Methylmaleinsäure, Fumarsäure, Aepfelsäure, Weinsäure, Zitronensäure, Benzoesäure, Zimtsäure, Mandelsäure, Salicylsäure, 4-Aminosalicylsäure, 2-Phenoxybenzoesäure, 2-Acetoxybenzoesäure, Embonsäure, Nicotinsäure oder Isonicotinsäure, ferner Aminosäuren, z.B. α-Aminosäuren, sowie Methansulfonsäure, Ethansulfonsäure, 2-Hydroxyethansulfonsäure, Ethan-1,2-disulfonsäure, Benzolsulfonsäure, 4-Methylbenzolsulfonsäure oder Naphthalin-2-sulfonsäure, oder mit anderen sauren organischen Verbindungen, wie Ascorbinsäure.

Die erfindungsgemässen Verbindungen der Formel $II_b$, die eine Carboxy-, Sulfo- oder Phosphogruppe tragen, können auch innere Salze oder Salze mit organischen Basen bilden. Bevorzugt sind Salze mit pharmazeutisch verwendbaren, nicht-toxischen Basen, beispielsweise mit physiologisch gut verträglichen organischen Aminen, z.B. mit tert-Butylamin, Hydroxyethylamin, Cyclohexylamin, Diethylamin, Di-n-Butylamin, Bis(hydroxyethyl)amin, Trimethylamin, Triethylamin, Tris(hydroxyethyl)amin oder Diethylhydroxyethylamin, ferner mit organischen Ammonium-Salzen, z.B. mit Tetramethylammonium oder Tetraethylammonium.

Zur Isolierung oder Reinigung können auch pharmazeutisch ungeeignete Salze Verwendung finden.

Die erfindungsgemässen Verbindungen der Formel $II_b$ und ihre pharmazeutisch verwendbaren Salze weisen wertvolle pharmakologische Eigenschaften auf. Beispielsweise sind Verbindungen der Formel $II_b$ in niedriger Konzentration von weniger als 0,3 µg/ml gegen verschiedene Keime wirksam, z.B. gegen Staphylococcus aureus, Bacillus subtilis, Micrococcus luteus und Escherichia coli. Neben antibiotischen Eigenschaften weisen die Verbindungen auch tumorhemmende Eigenschaften auf, bei spielsweise gegen die experimentellen Tumoren Leukämie L 1210/S2, menschliches Melanom SK-MEL 109, menschliches Lungenkarzinom MBA 9812, Colon Adenokarzinom 26 und Reticulum Zellsarkom M5076.

## Verfahren

Verfahren zur Herstellung von Verbindungen der Formel $II_b$ sind ebenfalls Gegenstand der Erfindung. Diese Verbindungen werden erhalten, indem man eine Verbindung der Formel II, worin A C=O oder C-OH bedeuten, acyliert und, wenn erwünscht, eine erfindungsgemässe Verbindung in eine andere erfindungsgemässe Verbindung überführt und/oder ein erhältliches Salz in die freie Verbindung oder ein anderes Salz oder eine erhältliche freie Verbindung in ein Salz überführt.

Unter Acylierung wird auch die Einführung einer Sulfogruppe (Acyl = $-SO_3H$) oder einer Phosphogruppe (Acyl = $-PO_3H_2$) verstanden.

Die Herstellung einer Verbindung der Formel $II_b$ oder eines Salzes davon erfolgt nach an sich bekannten Methoden, wie bei der Herstellung der Verbindungen der Formel I angegeben.

Die Erfindung betrifft auch diejenigen Ausführungsformen des Verfahrens, bei der man von einem auf irgendeiner Stufe des Verfahrens erhältlichen Zwischenprodukt ausgeht und die abschliessenden Schritte durchführt, das Verfahren auf irgendeiner Stufe abbricht und/oder eine nach dem erfindungsgemässen Verfahren erhältliche Verbindung in situ weiterverarbeitet.

## Pharmazeutische Präparate

Die Verbindungen der vorliegenden Erfindung und ihre pharmazeutisch verwendbaren Salze können z.B. zur Herstellung von pharmazeutischen Präparaten verwendet werden, welche eine wirksame Menge des Wirkstoffs vorzugsweise im Gemisch mit einer signifikanten Menge von anorganischen oder organischen, festen oder flüssigen, pharmazeutisch verwendbaren Trägerstoffen enthalten. Pharmazeutische Präparate, ihre Herstellung und Verwendung sind ebenfalls Gegenstand der vorliegenden Erfindung.

Die erfindungsgemässen pharmazeutischen Präparate eignen sich zur oralen oder bevorzugt parenteralen, z.B. intravenösen, intramusculären oder topischen Verabreichung.

Beispielsweise verwendet man die Wirkstoffe der Formel I oder $II_b$ der vorliegenden Erfindung in Form von injizierbaren, z.B. intravenös, intramuskulär, intradermal oder subcutan verabreichbaren Präparaten oder Infusionslösungen. Solche Lösungen sind vorzugsweise isotonische wässrige Lösungen oder Suspensionen, welche z.B. aus lyophilisierten Präparaten, welche den reinen Wirkstoff oder den Wirkstoff zusammen mit einem Trägermaterial, z.B. Dextran, Mannit oder Albumin enthalten, vor Gebrauch hergestellt werden können. Pharmazeutische Präparate zur oralen Anwendung sind gegebenenfalls sterilisiert und können Hilfsstoffe, z.B. Konservier-, Stabilisier-, Netz- und/oder Emulgiermittel, Löslichkeitsvermittler, Salze zur Regulierung des osmotischen Druckes und/oder Puffer enthalten. Geeignet sind auch entsprechende ölige Injektionssuspensionen, wobei man als Träger lipophile Lösungsmittel oder Vehikel, beispielsweise fette Oele, z.B. Sesamöl, Triglyceride oder synthetische Fettsäureester, z.B. Ethyloleat, verwendet. Die injizierbaren pharmazeutischen Präparate, die, wenn erwünscht, weitere pharmakologisch wertvolle Stoffe, z.B. andere Wirkstoffe, enthalten können, enthalten etwa 0,1 % bis 100 %, insbesondere etwa 1 % bis 50 %, im Falle von Lyophilisaten bis zu 100 %, des Wirkstoffes.

Geeignete Trägerstoffe für orale Präparate, z.B. Dragées, Tabletten oder Lacktabletten, sind insbesondere Füllstoffe, wie Zucker, z.B. Lactose, Saccharose, Mannit oder Sorbit, Cellulosepräparate und/oder Calciumphosphate, z.B. Tri-calciumphosphat oder Calciumhydrogenphosphat, Bindemittel, wie Stärkekleister auf der Basis z.B. von Mais-, Weizen-, Reis- oder Kartoffelstärke, Gelatine, Methylcellulose, Hydroxypropylmethylcellulose, Hydroxypropylcellulose und/oder Polyvinylpyrrolidon, und/oder, wenn erwünscht, Sprengmittel, wie die genannten Stärken, ferner Carboxymethylstärke, quervernetztes Polyvinylpyrrolidon, Agar, Alginsäure oder ein Salz davon, wie Natriumalginat oder niedermolekulare Carboxymethylcellulose. Hilfsmittel sind in erster Linie Fliessregulier-und Schmiermittel, z.B. Kieselsäure, Talk, Stearinsäure oder Salze davon, wie Magnesium- oder Calciumstearat, und/oder Polyethylenglykol.

Dragéekerne werden mit geeigneten, gegebenenfalls Magensaft-resistenten Ueberzügen versehen. Weitere oral anwendbare pharmazeutische Präparate sind Steckkapseln aus Gelatine sowie weiche, geschlossene Kapseln aus Gelatine und einem Weichmacher, wie Glycerin oder Sorbit. Als rektal anwendbare

pharmazeutische Präparate kommen z.B. Suppositorien in Betracht, welche aus einer Kombination des Wirkstoffs mit einer Suppositoriengrundmasse bestehen. Oral und rektal anwendbare Präparate enthalten etwa 0,1 % bis etwa 50 %, insbesondere etwa 1 % bis etwa 10 %, des Wirkstoffes und, wenn erwünscht, weitere pharmakologisch wertvolle Verbindungen.

Pharmazeutische Präparate für topische Anwendungen sind insbesondere Crèmen, Gele, Salben, Pasten, Schäume, Tinkturen und Lösungen, die von etwa 0,05 % bis etwa 10 %, insbesondere von etwa 0,5 % bis etwa 5 % des Wirkstoffes enthalten.

Die pharmazeutischen Präparate werden in an sich bekannter Weise, z.B. mittels konventioneller, in Pharmakopöen beschriebener Lösungs-, Suspendier-, Misch- oder Lyophilisierungsverfahren, hergestellt.

Die Erfindung betrifft ebenfalls die Verwendung der erfindungsgemässen Verbindungen der Formel I oder von Salzen davon als Heilmittel, beispielsweise in Form von pharmazeutischen Präparaten, zur Behandlung von bakteriellen Infektionen durch gram-positive Keime und zur Behandlung von Tumoren, insbesondere Lungen- und Magen/Darm-Tumoren und Leukämie, bei Warmblütern, z.B. Menschen und Säugetieren, durch enterale, z.B. orale, oder bevorzugt parenterale Applikation von therapeutisch wirksamen Dosen.

Die zur Anwendung gelangenden täglichen Dosen betragen je nach Spezies, Alter, individuellem Zustand, Schwere der Erkrankung und Applikationsweise entsprechend der Beurteilung des behandelnden Arztes zwischen etwa 0,01 mg und etwa 10 mg, insbesondere zwischen etwa 0,1 mg und etwa 1 mg pro kg Körpergewicht.

Die nachfolgenden Beispiele illustrieren die Erfindung, schränken deren Umfang jedoch in keiner Weise ein.

Beispiel 1: Bakterium Myxococcus xanthus Mx x48

a) Herkunft und Zugänglichkeit des Produktionsstamms:

Das Bakterium Myxococcus xanthus Stamm Mx x48, Familie Myxococcaceae, Ordnung Myxobacterales, wurde im Mai 1980 aus einer Bodenprobe der Oase Gabès, Tunesien, isoliert. Der Stamm Mx x48 ist bei der National Collection of Industrial and Marine Bacteria, Aberdeen, Schottland, am 26. Mai 1986 unter der Nummer NCIB 12 268 für Patentzwecke nach dem Budapester Vertrag hinterlegt worden.

b) Beschreibung des Produktionsstamms:

Vegetative Stäbchen, zylindrisch mit kurz verjüngten Enden, zigarrenförmig, 0,8 x 3,5-5 µm, bewegen sich auf Oberflächen kriechend-gleitend. Auf geeigneten Medien, z.B. VY/2-Agar (s. unten), bildet der Organismus "Fruchtkörper" von 50-150 µm Durchmesser in Form von intensiv rotorangen bis blass grauen weichschleimigen Tropfen, Pusteln oder Köpfchen auf der Agaroberfläche. In deren Inneren finden sich kugelige, stark lichtbrechende Myxosporen von 1,8-1,9 µm Durchmesser. Die Kolonien tendieren dazu, sich hauchartig als "Schwärme" auf der Agaroberfläche auszubreiten und können bis zu mehreren cm gross werden. Auf manchen Medien sind sie intensiv grünlich-gelb bis orange gefärbt.

c) Kulturbedingungen:

Der Organismus wächst gut auf Peptonagar, z.B. CY-Agar (Casitone [Difco] 0,3 %, Hefeextrakt [Difco] 0,1 %, $CaCl_2 \cdot 2H_2O$ 0,1 %, Agar 1,5 %, pH 7,2) oder auf Hefeagar, z.B. VY/2-Agar (Backhefe 0,5 % bezogen auf Frischgewicht, $CaCl_2 \cdot 2H_2O$ 0,1 %, Agar 1,5 %, pH 7,2). In Flüssigmedien wächst Mx x48 in homogener Zellsuspension sowohl in Schüttelkolben (bei etwa 160 upm) als auch in Bioreaktoren. Als Kulturmedium ist z.B. geeignet MDI 1.m. (Pepton aus Casein, tryptisch verdaut [Merck, Darmstadt] 0,3 %, $MgSO_4 \cdot 7H_2O$ 0,2 %, $CaCl_2 \cdot 2H_2O$ 0,05 %, pH 7,2). Mx x48 ist streng aerob. Alle Kulturen werden bei 30°C gehalten. Die Generationszeit in MDI 1.m. liegt bei etwa 6,5 Stunden ($\mu$ = 0,15 pro Stunde). Die maximal erreichte optische Dichte (623 nm, 1 cm Weglänge, Eppendorf Photometer) liegt bei 1,3 bis 1,5. Die Konzentration des Peptons aus Casein kann auch erhöht werden, z.B. auf 0,5 oder 1 %. Die Generationszeit ändert sich dabei nicht, dagegen erhöht sich die optische Dichte auf ca. 2 bzw. 3,5. Die Antibiotikumproduktion wird bei steigen der Pepton-Konzentraton aber geringer im Vergleich zur Produktion in MDI 1.m. Als Kulturmedien, in denen der Stamm unter Antibiotikumbildung wächst, sind ferner folgende Medien geeignet: 0,05 % Casitone, 0,2 % $MgSO_4 \cdot 7H_2O$ und 0,05 % $CaCl_2 \cdot 2H_2O$, pH 7,2, enthaltend entweder 0,5 % Probion S (Einzellerprotein der Fa. Hoechst) oder 0,5 % Maisquellwasser oder 0,5 % Protaminal (Fischproteinkonzentrat der Fa. Asta) oder 0,5 % Hefeextrakt oder 0,3 % Casamino acids (Difco).

d) Konservierungsmöglichkeiten:

1. Durch Einfrieren vegetativer Zellen von Platten- oder Flüssigkulturen in Pepton-Lösung bei -80°C (mehrere Jahre lebensfähig). 2. Durch Trocknen von Fruchtkörpern auf Filterpapier (mehrere Jahre bei Zimmertemperatur lebensfähig). 3. Durch Trocknen von Fruchtkörpern in Milch, Lagerung in zugeschmolzenen Ampullen unter Stickstoff bei Zimmertemperatur (mehrere Jahre lebensfähig). 4. Durch Suspendieren vegetativer Zellen von Platten oder Flüssigkulturen in Peptonlösung und Einfrieren in flüssigem Stickstoff (mehrere Jahre lebensfähig).

Beispiel 2: Herstellung von Zellüberständen enthaltend Saframycin Mx 1

a) Vorkultur:
50 ml MDI 1.m. werden mit 0,5 ml Zellsuspension angeimpft und bei 30° C und 160 upm über 2 Tage gehalten.

b) Vorfermentation:
Ein 25 l-Bioreaktor der Firma Braun, Melsungen, mit Blattrührsystem wird mit Medium MDI 1.m. gefüllt und mit der Vorkultur a) beimpft. Die Temperatur wird bei 30°C gehalten. Die Umdrehungsgeschwindigkeit des Rührers beträgt 200 upm, die Belüftung 0,1 $Nm^3/h$. Ohne weitere Veränderungen wird die Fermentation über 66 Stunden durchgeführt. Nach dieser Zeit ist eine OD (623 nm) von 1,1 und ein pH von 7,9 erreicht.

c) Hauptfermentation:
Ein Bioreaktor mit 700 l Inhalt, Firma Giovanola, Monthey, Schweiz, mit Blattrührsystem wird mit Medium MDI 1.m. gefüllt. Nach dem Beimpfen mit der Vorfermentation b) wird eine Umdrehungsgeschwindigkeit von 150 upm und eine Belüftung von 1 $Nm^3/h$ eingestellt. Durch Erhöhung der Belüftung und der Drehzahl wird der Sauerstoffpartialdruck im Verlaufe der Kultivierung oberhalb von 60 % Sättigung gehalten. Die Fermentation wird durch regelmässige probenentnahme, Aufarbeitung der Proben nach Beispiel 3 und Analyse mit HPLC überwacht und abgebrochen, sobald die Bildung von Saframycin Mx 1 und Mx 1 BC nicht mehr weiter ansteigt. Nach 50 Stunden ist eine OD von 1,3 und ein pH von 8 erreicht. Im Agardiffusionstest gegen Staph. aureus erhält man einen gerade noch sichtbaren Hemmhof (ca. 7 mm Durchmesser), wenn man den Kulturüberstand 1:64 verdünnt.

Beispiel 3: Isolierung und Reinigung von Saframycin Mx 1 (1) und Mx 1 BC (1-BC)

a) Aufarbeitung einer 700 Liter Fermentation:
Gegen Ende der Fermentation von Beispiel 2 c) nach ca. 40 Stunden werden 4,0 Liter XAD-Harz 1180® (Fa. Röhm & Haas) in die Kulturbrühe eingebracht. Das Gemisch wird 10 Stunden unter langsamen Rühren weiter fermentiert. Das Harz wird über ein Sieb von den Zellen, in denen sich kein Antibiotikum befindet, und vom Medium abgetrennt. Es wird in eine Chromatographiesäule überführt und in einem Zuge mit 12 Litern (1 Liter/Std.) Isopropanol eluiert. Das Antibiotikum wird mit den ersten 6 Litern Isopropanol zu über 90% eluiert. Die Saframycine enthaltenden Fraktionen werden sofort mit 0,05 % (v/v) Essigsäure versetzt und am Rotationsverdampfer in braunen Kolben bei Temperaturen bis zu höchstens 30°C auf 1-2 l (pH 4,2-4,5) eingeengt, dann mit Essigester gewaschen. Der eingeengte XAD-Extrakt enthält etwa 22 g Feststoffe.

Die Saframycine sind basische, licht- und oxidationsempfindliche Hydrochinon/Chinon-Derivate, die nur bei pH-Werten kleiner als 7 stabil sind. Die Reindarstellung erfolgt somit weitgehend unter Lichtausschluss und bei pH-Werten um 5. Die Substanz-haltigen Fraktionen werden in braun eingefärbten Glasbehältern zwischengelagert, die Langzeitlagerung geschieht bei -70°C.

b) Reinigung der Saframycine:
Der Rohextrakt wird in 300 ml 0,07 M Phosphatpuffer pH 5 gelöst und auf eine offene Säule aufgetragen, die mit 1 Liter gequelltem CM-Sephadex® C-25 der Fa. Pharmacia in 0,07 M Phosphatpuffer pH 5 gefüllt ist. Zunächst wird mit 2 Liter Phosphatpuffer alles nicht absorbierte Material eluiert. Danach wird bei einem Fluss von 4 Liter pro Stunde mit 0.07 M Phosphatpuffer pH 5 und einem Natrium chlorid-Gradienten, der innerhalb von 3 Stunden von 0 bis 0,5 M Natriumchlorid geführt wird, Saframycin Mx 1 und Saframycin Mx 1 BC von der Säule eluiert, was durch eine wandernde gelbliche Bande sichtbar wird. Das Eluat wird in einem By-pass mit einem Photometer bei 277 nm fortlaufend überwacht. In einer ersten Fraktion werden 1,2 g reines Saframycin Mx 1, in einer zweiten Fraktion 1,8 g eines 1:1-Gemisches von Saframycin Mx 1 und Mx 1 BC (Bis-chinon) erhalten. Diese Fraktionen enthalten noch Reste an Phosphat-Salz. Zur Entsalzung wird auf eine XAD-2®-Säule aufgetragen, mit 2 Bettvolumina Wasser gewaschen und mit Methanol eluiert. Statt über XAD-2 kann auch über eine Sephadex® LH-20 Säule mit Methanol und 1 % Ameisensäure oder Essigsäure als Laufmittel chromatographiert werden, wobei Phosphat durch Formiat beziehungsweise Acetat ausgetauscht wird. Das gereinigte Produkt wird mit HPLC (Nucleosil® C-18, 7,5 µm, Methanol/ Wasser 60:40/0,005 M Heptansulfonsäure oder HD-Sil® 18, 7 µm, Methanol/Phosphatpuffer pH 6,5 60:40) analytisch untersucht und enthält nur noch Saffiramycin Mx 1 bzw. ein Gemisch von Saframycin Mx 1 und dem Bis-chinon-Derivat Saframycin Mx 1 BC.

Wird mit der Aufarbeitung einer Fermentationsbrühe zu lange zugeartet (Zugabe des Adsorberharzes erst nach 50 oder mehr Stunden), so wandeln sich Saframycin Mx 1 und Mx 1 BC langsam in Saffiramycin Mx 2 und Mx 2 BC um, d.h. in Verbindungen der Formel I, worin $R^1$ und $R^2$ Wasserstoff und A C-OH beziehungsweise C=O bedeuten. Das Gemisch der Saframycine kann durch semi-präparative HPLC einer RP-18 Säule an (HD-Sil® 18 Fa. Organogen) mit Methanol:Wasser 1:1 und 0,5 % Triethylammoniumformiatpuffer pH 6,0 aufgetrennt werden.

Beispiel 4: 21-Cyano-Saframycin Mx 1 (2) und 21-Cyano-Saframycin Mx 1 BC (2-BC)
20 mg Saframycin Mx 1 (1) werden in 2 ml Phosphatpuffer pH 5,5 (0,1 M) gelöst, und mit 5 mg Natriumcyanid (gelöst im gleichen Puffer) versetzt. Unter Stickstoff wird bei Raumtemperatur für 15 Minuten gerührt. Das HPLC-Diagramm unter Standardbedingungen (HD-Sil® 18, 7 µm, Laufmittel Methanol:Phosphat-Puffer (0,06 M, pH 6,5) = 60:40, Fluss 1,5 ml/min) zeigt nach dieser Zeit bereits quantitative Umsetzung an

(Retentionszeiten $R_t$(1) = 3,03 min, $R_t$(1-BC) = 4,12 min, $R_t$(2) = 3,61 min, $R_t$(2-BC) = 6,0 min).

Das gesamte Reaktionsgemisch wird auf eine XAD-2®-Säule (2,5 cm x 10 cm) aufgetragen und mit zwei Bettvolumina Wasser gespült, womit überschüssige Salze entfernt werden. Danach wird mit einem Bettvolumen Methanol eluiert und das Eluat gefriergetrocknet, wobei 21-Cyano-Saframycin Mx 1 (2) zurückbleibt.

$^1$H-NMR in $CD_3OD$: δ = 4,02 ppm (s, 7-OMe), 3.73 (s, 17 OMe), 3,66 (s, 14-OMe), 2,40 (s, N-Me), 2,20 (s, 16-Me), 1,92 (s, 6-Me), 0,74 (d, 25-Me).

Wird die Reaktion nicht unter peinlichem Ausschluss von Luftsauerstoff durchgeführt, so entsteht gleichzeitig das entsprechende Bis-chinon 2-BC. Höherer pH (pH 7) und längere Reaktionszeiten (45 statt 15 Min.) begünstigen die Bildung des Bis-chinons 2-BC.

Das Verhältnis von 2 zu seeinem Bischinon 2-BC lässt sich somit wie gewünscht steuern. Die Trennung beider Substanzen erfolgt mittels Mitteldruckchromatographie an Umkehrphase (reversed phase) Kieselgel (HD-Sil® 18, Laufmittel Methanol:Phosphat-Puffer H 6,5 = 70:30).

21-Cyano-Saframycin Mx 1 BC (2-BC):

UV: $\lambda_{max}$ 264 nm (1g ε = 4,31, in Methanol). - IR(Film): ν = 1685, 1655, 1615 $cm^{-1}$. - $^{13}$C-NMR($CD_3OD$), Formiat statt Phosphat: δ = 188,0 ppm (s, C-15), 186,8 (s, C-5), 184.2 (s, C-18), 182,7 (s, C-8), 172,0 (s, C-23), 168,1 (s, Formiat), 157,2 (s, C-7), 156,7 (s, C-17), 143,0 (s, C-9), 142,5 (s, C-19), 137,8 (s, C-10), 136,7 (s, C-20), 130,5 (s, C-16), 129,3 (s, C-6), 117,9 (s, -CN), 71,0 (d, C-14), 61,5 (2q, 7- und 17-$OCH_3$), 61,3 (d, C-21), 59,7 q, 14-$OCH_3$), 58,3 und 58,2 (d, C-13 oder C-3), 55,7 und 54,9 (2d, C-11 oder C-1), 50,1 (d, C-24), 42,8 (t, C-22), 42,2 (q, N-$CH_3$), 26,7 (t, C-4), 18,0 (q, $CH_3$-25), 8,8 (2q, C-6 und C-16). - $^1$H-NMR($CD_3OD$): δ = 4,70 ppm (d, J = 2,5 Hz [13-21], 1H, 21-H), 4,16 (dd, J = 3 Hz [3-11], J = 1,2 Hz [11-13], 1H, 11-H), 4,10 (s, 1H, 14-H), 4,04, (s, 3H, 17-$OCH_3$), 4,01 (s, 3H, 7-$OCH_3$), 3,95 (m, 1H, 1-H, Cross-peaks im 2D-COSY mit 22a-H, 22b-H und 4b-H), 3,77 (q, J = 7,2 Hz [24-(25-Me)], 1H, 24-H), 3,60 (m, 1H, 22a-H, Cross-peaks mit 1-H und 22b-H), 3,60 (m, 1H, 13-H, Cross-peak mit 21-H und 11-H), 3,59 (s, 3H, 14-$OCH_3$), 3,45 (dd, J = 3,5 Hz [1-22b], J = 14,0 Hz [22a-22b], 1H, 22b-H), 2,93 (ddd, J = 12 Hz [3-4b], J = 2 Hz [3-4a], J = 3 Hz [3-11], 1H, 3-H), 2,83 (dd, J = 17 Hz [4a-4b], J = 2 Hz [3-4a], 1H, 4-$H_a$), 2,50 (s, 3H, N-$CH_3$), 2,00 (s, 3H, 16-$CH_3$), 1,95 (s, 3H, 6-$CH_3$), 1,34 (m, 1H, 4-$H_b$, Cross-Peaks mit 4-$H_a$, 3-H und 1-H), 1,21 (d, J = 7,2 Hz, 3H, 24-$CH_3$). - FAB-MS (Pos.Ionen): m/z = 598 $(M+H)^+$, m/z = 496 $[M-CH_2NHCOCH(CH_3)NH_3]^+$ (Verlust der Seitenkette an C-1). Hochauflösung: Gef. 598,2851, für $C_{30}H_{40}N_5O_8$ ber. 598,2877; gef. 496,2073, für $C_{26}H_{30}N_3O_7$ ber. 496,2084.

Beispiel 5: N-Acyl-21-cyano-Saframycin Mx 1-Derivate

Allgemeine Arbeitsvorschrift:

In einer Stickstoffatmosphäre werden 50 mg Saframycin Mx 1 (1) in 3 ml Phosphatpuffer pH 6,5 gelöst, mit 10 mg Natriumcyanid versetzt und 10 Min. bei Raumtemperatur gerührt. Danach wird mit 1N NaOH auf pH - 7,5 eingestellt und mit 3 ml Methanol verdünnt. Zu dieser Lösung werden 1-2 Moläquivalente des entsprechenden Acylierungsreagenses als Carbonsäure-Anhydrid gelöst in 5 ml Tetrahydrofuran zugetropft. Die Reaktionsmischung wird 30 Minuten gerührt. Gemäss HPLC (Standardbedingungen von Beispiel 4) ist je nach Acylierungsmittel das Ausgangsmaterial Mx 1 nach 5-20 Minuten vollständig verbraucht. Der pH der Reaktionslösung wird ständig überwacht und kontinuierlich mit 1N NaOH auf pH 7,5 gehalten. Das gesamte Reaktionsgemisch wird nach Beendigung der Umsetzung auf eine XAD-2®-Säule (2,5 cm x 10 cm) aufgetragen. Die Säule wird mit zwei Bettvolumina Wasser gespült. Danach wird das gewünschte Produkt mit Methanol eluiert. Das Methanol wird im Vakuum abdestilliert und das acylierte Saframycin durch Abfiltrieren vom wässrigen Rückstand oder durch Gefriertrocknung isoliert.

Wenn Luftsauerstoff zur Lösung tritt, wird nach dieser Vorschrift neben N-Acyl-21-cyano-Saframycin Mx 1 gleichzeitig auch N-Acyl-21-cyano-Saframycin Mx 1 BC (das Bis-chinon) gebildet. Die beiden Verbindungen werden durch semipräparative HPLC an reversed phase Kieselgel (HD-Sil® 18) getrennt.

Die als Acylierungsreagentien verwendeten Carbonsäure-Anhydride sind kommerziell erhältlich oder werden folgendermassen hergestellt: 1 mMol Carbonsäure in 5 ml Tetrahydrofuran wird mit 0,5 mMol Dicyclohexylcarbodiimid versetzt und bei Raumtemperatur 30 Minuten gerührt. Der ausgefallene Dicyclohexylharnstoff wird abfiltriert. Das Filtrat, das das gewünschte Carbonsäure-Anhydrid enthält, wird als Lösung direkt zu Saframycin Mx 1 (1) in Phosphatpuffer getropft.

Anstelle von (1) kann auch das 21-Cyano-Saframycin Mx 1 (2) acyliert werden. Man verwendet dieselbe Arbeitsvorschrift, lässt jedoch die Behandlung mit Natriumcyanid weg.

Nach dieser Arbeitsvorschrift wurden beispielsweise die folgenden Verbindungen hergestellt:

a) N-Acetyl-21-cyano-Saframycin Mx 1 (3):

$^1$H-NMR in $CD_3OD$: 4,0 ppm (s, 7-OMe), 3,72 (s, 17-OMe), 3,68 (s, 14-OMe), 2,42 (s, N-Me), 2,20 (s, 16-Me), 1,93 (s, N-Acetyl), 1,91 (s, 6-Me), 0,72 (d, 25-Me), -HPLC, Standardbedingungen: $R_t$ = 3,17 min.

b) N-Acetyl-21-cyano-Saframycin Mx 1 BC (3-BC):

$^1$H-NMR in $CD_3OD$: 4,08 ppm (s, 7-OMe), 4,06 (s, 17-OMe), 3,60 (s, 14-OMe), 2,50 (s, N-Me), 1,98 (s, 16-Me), 1,92 (s, 6-Me), 1,75 (s, N-Acetyl), 1,13 (d, 25-Me), -FAB-MS: Gef. 635,2588, für $C_{32}H_{37}N_5O_9$ ber. 635,2592, $(M^-)$. - HPLC, Standardbedingungen: $R_t$ = 5,32 min.

c) N-Caproyl-21-cyano-Saframycin Mx 1 (4):
$^1$H-NMR in $CD_3OD$: 4,01 (s, 7-OMe), 3,71 (s, 17-OMe), 3,67 (s, 14-OMe), 2,42 (s, N-Me), 2,20 (s, 16-Me), 2,20 (t, Caproyl-α-$CH_2$), 1,92 (s, 6-Me), 1,58 (t, Caproyl-β-$CH_2$), 1,35 (m, Caproyl-γ,δ-$CH_2$), 0,95 (t, Caproyl-Me), 0,72 (d, 25-Me). - FAB-Ms: Gef. 694,3411, für $C_{36}H_{48}N_5O_9$ ber. 694,3452, $(M-H)^-$ des Bishydrochinons. - HPLC, Standardbedingungen: $R_t$ = 6,16 min.

d) N-Caproyl-21-cyano-Saframycin Mx 1 BC (4-BC):
$^1$H-NMR in $CD_3OD$: 4,07 (s, 7-OMe), 4,04 (s, 17-OMe), 3,60 (s, 14-OMe), 2,50 (s, N-Me), 2,39 (t, 2H, Caproyl-α-$CH_2$), 1,69 (m, Caproyl-β-$CH_2$), 1,37 (m, Caproyl-γ,δ-$CH_2$), 1,10 (d, 25-Me), 0,93 (t, Caproyl-Me). - HPLC, Standardbedingungen: $R_t$ = 15,44 min.

e) N-Benzoyl-21-cyano-Saframycin Mx 1 (5):
FAB-MS: Gef. 697,2762, für $C_{37}H_{39}N_5O_9$ ber. 697,2748, $(M^-)$ des Bishydrochinons. -HPLC, Standardbedingungen: $R_t$ = 5,16 min.

f) N-Benzoyl-21-cyano-Saframycin Mx 1 BC (5-BC):
$^1$H-NMR in $CD_3OD$: 7,4-7,8 (m, Phenyl), 3,99 (s, 17-OMe), 3,97 (s, 7-OMe), 3,61 (s, 14-OMe), 2,50 (s, N-Me), 1,92 (s, 6-Me), 1,68 (s, 16-Me), 1,28 (d, 25-Me). -HPLC, Standardbedingungen: $R_t$ = 9,22 min.

g) N-Pivaloyl-21-cyano-Saframycin Mx 1 (6):
$^1$H-NMR in $CDCl_3$: 3,95 (s, 7-OMe), 3,68 (s, 17-OMe), 3,60 (s, 14-OMe), 2,46 (s, N-Me), 2,15 (s, 16-Me), 1,82 (s, 6-Me), 1,07 (s, Pivaloyl-Me), 0,61 (d, 25-Me). - FAB-MS: Gef. 677,3057 für $C_{35}H_{43}N_5O_9$ ber. 677,3061, $(M^-)$ des Bishydrochinons. - HPLC Standardbedingungen: $R_t$ = 4,54 min.

h) N-Pivaloyl-21-cyano-Saframycin Mx 1 BC (6-BC):
$^1$H-NMR in $CD_3OD$: 3,99 (s, 17-OMe), 3,95 (s, 7-OMe), 3,52 (s, 14-OMe), 2,35 (s, N-Me), 1,89 (s, 6- oder 16-Me), 1,79 (s, 6- oder 16-Me), 1,10 (d, 25-Me), 1,07 (s, Pivaloyl-Me), - HPLC, Standardbedingungen: $R_t$ = 9,79 min.

i) N-(3-Carboxypropionyl)-21-cyano-Saframycin Mx 1 (7),
hergestellt mit Bernsteinsäureanhydrid: $^1$H-NMR in $CD_3OD$: 4,03 (s, 7-OMe), 3,71 (s, 17-OMe), 3,65 (s, 14-OMe), 2,7-2,3 (m, 4H, Succinyl-$CH_2$), 3,45 (s, N-Me), 2,34 (s, 16-Me), 1,89 (s, 6-Me), 0,70 (d, 25-Me). -FAB-MS: Gef. 693,2654, für $C_{34}H_{39}N_5O_{11}$ ber. 693,32646 $(M-H)^-$ des Bishydrochinons als Triethylammonium-Salz. - HPLC, Standardbedingungen: $R_t$ = 2,06 min.

j) N-(3-Carboxypropionyl)-21-cyano-Saframycin Mx 1 BC (7-BC):
$^1$H-NMR in $CD_3OD$: 4,08 (s, 7-OMe), 4,06 (s, 17-OMe), 3,60 (s, 14-OMe), 2,50 (s, N-Me), 2,36 (m, 2H, Succinyl-$CH_2$), 2,25 (t, 2H, Succinyl-$CH_2$), 1,97 (s, 16-Me) 1,90 (s, 6-Me), 1,14 (d, 25-Me). - HPLC, Standardbedingungen: $R_t$ = 3,83 min.

k) N-(O-Benzyl-N-benzyloxycarbonyl-β-aspartyl)-21-cyano-Saframycin Mx 1
[N-(3-Benzyloxycarbonyl-3-benzyloxycarbonylamino-propionyl)-21-cyano-Saframycin Mx 1] (8):
$^1$H-NMR in $CD_3OD$: 7,37 (s, breit, 2 x Phenyl), 5,19 (d, Benzyl-$CH_2$), 5,14 (s, Benzyl-$CH_2$), 4,57 (dd, Aspartyl-α-CH), 3,98 (s, 7-OMe), 3,67, (s, 17-OMe), 3,60 (s, 14-OMe), 2,74 (dddd, Aspartyl-β-$CH_2$), 2,41 (s, N-Me), 2,16 (s, 16-Me), 1,88 (s, 6-Me), 0,70 (d, 25-Me). - FAB-MS: Gef. 932,3592, für $C_{49}H_{52}N_6O_{13}$ ber. 932,3592 $(M^-)$. - HPLC, Standardbedingungen: $R_t$ = 11,7 min.

l) N-(O-Benzyl-N-benzyloxycarbonyl-β-aspartyl)-21-cyano-Saframycin Mx 1 BC (8-BC):
$^1$H-NMR in $CD_3OD$: 7,37 (s, breit, 2 x Phenyl), 5,16 (d, Benzyl-$CH_2$), 5,13 (s, Benzyl-$CH_2$) 4,45 (t, Aspartyl-α-CH), 4,00 (s, 17-OMe), 3,99 (s, 7-OMe), 3,57 (s, 14-OMe), 2,56 (d, Aspartyl-β-$CH_2$), 2,47 (s, N-Me), 1,91 (s, 16-Me), 1,86 (s, 6-Me), 1,10 (d, 25-Me). - HPLC, Standardbedingungen: $R_t$ = 35,9 min.

Beispiel 6: N-Formyl-21-cyano-Saframycin Mx 1 (9)

Zu einer auf -30°C gekühlten Lösung von 50 mg 21-Cyano-Saframycin Mx 1 (2) in 5 ml Pyridin werden nacheinander 160 mg Ameisensäure, 340 mg Acetanhydrid und 28 mg p-N,N-Dimethylaminopyridin (DMAP) zugesetzt. Es wird für 30 Min. bei gleicher Temperatur gerührt. Danach wird bei Oelpumpenvakuum die gesamte Reaktionslösung eingeengt und über Mitteldruck-reversed phase-Chromatographie mit Methanol:Phosphatpuffer pH 6,5 = 1:1 und einem Fluss von 12,5 ml/min chromatographiert. Nach Entsalzung über XAD-2 wird die reine Titelverbindung 9 erhalten. $^1$H-NMR ($CD_3OD$): 7,95 (s, Formyl-H), 4,0 (s, 7-Ome), 3,72 (s, 17-Ome), 3,66 (s, 14-Ome), 2,41 (s, N-Me), 2,18 (16-Me), 1,91 (s, 6-Me), 0,78 (d, 25-Me). - FAB-MS: Gef. 623,2580, für $C_{31}H_{37}N_5O_9$ ber. 623,2591, $(M)^-$. - HPLC, Standardbedingungen: $R_t$ = 2,79 min.

Das gleichzeitig gebildete und bei der Chromatographie abgetrennte N-Formyl-21-cyano-Saframycin Mx 1 BC (9-BC) weist im HPLC eine Retentionszeit von 4,38 min unter Standardbedingungen auf.

Beispiel 7: N-Phenylamino-carbonyl- und -thiocarbonyl-21-cyano-Saframycin Mx 1 (10) und (11)

50 mg 21-Cyano-Saframycin Mx 1 (2) werden in einem 1:1 Gemisch aus Phosphatpuffer (pH 7,8) und Tetrahydrofuran mit 1,05 Aequivalenten Phenylisocyanat bzw. Phenylisothiocyanat unter Stickstoffatmosphäre versetzt. Das Gemisch wird bei Raumtemperatur (Phenylisocyanat) bzw. bei 50°C (Phenylisothiocyanat) 30 Minuten gerührt. Die Reaktion wird mittels HPLC (Bedingungen siehe Beispiel 4) verfolgt. Die abgekühlte Lösung wird zwischen Essigsäureethylester und Wasser verteilt und über reversed phase Kieselgel chromatographiert. Bei dieser Chromatographie wird gleichzeitig das als Nebenprodukt entstandene Bis-chinon abgetrennt.

a) N-Phenylaminocarbonyl-21-cyano-Saframycin Mx 1 (10):

$^1$H-NMR in $CDCl_3$: 7,0-7,35 (m, Phenyl), 6,63 (s, NH), 6,31 (t, NH), 5,07 (d, NH), 3,98 (s, 17- oder 7-OMe), 3,96 (s, 17- oder 7-OMe), 3,51 (s, 14-OMe), 2,49 (s, N-Me), 1,96 (s, 6-Me), 1,76 (s, 16-Me), 1,10 (d, 25-Me). -FAB-MS: Gef. 712,2850, für $C_{37}H_{40}N_6O_9$ ber. 712,2856, ($M^-$). - HPLC, Standardbedingungen: $R_t$ = 10,50 min.

b) N-Phenylamino-thiocarbonyl-21-cyano-Saframycin Mx 1 (11):

$^1$H-NMR in $CD_3OD$: 7,2-7,5 (m, Phenyl), 3,99 (s, 7-OMe), 3,73 (s, 17-OMe), 3,63 (s, 14-OMe), 2,38 (s, N-Me) 2,13 (s, 6-Me), 1,89 (s, 16-Me), 0.84 (d, 25-Me). - FAB-MS: Gef. 728,2616, für $C_{37}H_{40}N_6O_8S$ ber. 728,2628, ($M^-$). - HPLC, Standardbedingungen: $R_t$ = 4,75 min.

c) N-Phenylamino-thiocarbonyl-21-cyano-Saframycin Mx 1 BC (11-BC):

$^1$H-NMR in $CD_3OD$: 7,2-7,5 (m, Phenyl), 4,06 (s, 17-OMe), 4,00 (s, 7-OMe), 3,59 (s, 14-OMe), 2,49 (s, N-Me), 1,95 (s, 6-Me), 1,81 (s, 16-Me), 1,21 (d, 25-Me). - HPLC, Standardbedingungen: $R_t$ = 11,44 min.

Beispiel 8: N,O,O-Triacetyl-21-cyano-Saframycin Mx 1 (12):

Die phenolischen OH-Gruppen des Hydrochinon-Teiles werden wie folgt acyliert: 20 mg N-Acetyl-21-cyano-Saframycin Mx 1 (3) werden in 1 ml Pyridin gelöst und mit 0,5 ml Acetanhydrid versetzt. Die Mischung wird 60 Minuten bei Raumtemperatur gerührt. Am Oelpumpenvakuum wird das überschüssige Reagens und Lösungsmittel entfernt. Der Rückstand wird über Mitteldruck-Chromatographie (HD-Sil® 18, Methanol:Phosphatpuffer pH 6,5 = 60:40) gereinigt. Neben der Titelverbindung 12 werden geringe Mengen Saframycin Mx 1 BC (1-BC) isoliert. $^1$H-NMR in $CD_3OD$: 4,06 (s, 7-OMe), 3,78 (s, 17-OMe), 3,56 (s, 14-OMe), 2,49, (3 x s, N-Me, 2 x O-Acetyl), 2,16 (s, 16-Me), 1,88 (s, 6-Me), 1,72 (s, N-Acetyl), 1,02 (d, 25-Me). - FAB-MS: Gef. 721,2971, für $C_{36}H_{43}N_5O_{11}$ ber. 721,2960, ($M^-$). - HPLC, Standardbedingungen: $R_t$ = 3,55 min.

Beispiel 9: N-p-Toluolsulfonyl-21-cyano-Saframycin Mx 1 (13):

50 mg 21-Cyano-Saframycin Mx 1 (2) werden in Pyridin gelöst, auf 0°C abgekühlt und unter Stickstoffatmosphäre mit äquimolarer Menge Toluolsulfochlorid versetzt. Man lässt während 30 Minuten auf Raumtemperatur aufwärmen, verteilt zwischen Essigsäureethylester und Wasser, trocknet die Essigester-Phase und chromatographiert den Rückstand auf reversed phase Kieselgel mit Methanol:Puffer pH 6,5 = 60:40.

$^1$H-NMR in $CD_3OD$: 7,64 (d, Phenyl), 7,38 (d, Phenyl), 4,04 (s, 7-OMe), 3,69 (s, 17-OMe), 3,63 (s, 14-OMe), 2,48 (s, Tolyl-Me), 2,31 (s, N-Me), 2,15 (s, 16-Me), 1,92 (s, 6-Me), 0,69 (d, 25-Me). - FAB-MS: Gef. 750,2758, für $C_{37}H_{44}N_5SO_{10}$ ber. 750,2808. - HPLC, Standardbedingungen: $R_t$ = 5,97 min.

Als Nebenprodukt wird bei der Chromatographie N-p-Toluolsulfonyl-21-cyano-Saframycin Mx 1 BC (13-BC) abgetrennt, $R_t$ = 9,47 min bei HPLC-Standardbedingungen.

Beispiel 10: N-Sulfo-21-cyano-Saframycin Mx 1 und Mx 1 BC (14) und (14-BC):

80 mg 21-Cyano-Saframycin Mx 1 (2) werden in 3 ml Methanol und 3 ml Phosphatpuffer pH 7,8 (0,12 M) gelöst und mit der halben äquivalenten Menge an Pyridin-Schwefeltrioxid-Komplex versetzt. Mit 1N NaOH wird der pH auf den Ausgangswert 7,8 eingestellt. Danach wird nochmals die gleiche Menge Pyridin-Schwefeltrioxid zugesetzt. Nach 10 Min. ist die Reaktion beendet. Die Lösung wird im Oelpumpenvakuum eingeengt. Die Titelverbindungen werden über reversed phase-Mitteldruckchromatographie voneinander getrennt und als Triethylammoniumsalze isoliert.

a) N-Sulfo-21-cyano-Saframycin Mx 1 (14):

$^1$H-NMR ($CD_3OD$): 4,06 (s, 7-OMe), 3,74 (s, 17-OMe), 3,64 (s, 14-OMe), 3,23 (q, 3x $CH_2$, Triethylammonium-$CH_2$), 2,46 (s, N-Me), 2,27 (s, 16-Me), 1,88 (s, 6-Me), 1,34 (t, 3x $CH_3$, Triethylammonium-$CH_3$), 0,73 (d, 25-Me). - FAB-MS: Gef. 674,2101, für $C_{30}H_{36}N_5SO_{11}$ ber. 674,2131, $(M-H)^-$. - HPLC, Standardbedingungen: $R_t$ = 1,82 min.

b) N-Sulfo-21-cyano-Saframycin Mx 1 BC(14-BC):

$^1$H-NMR ($CD_3OD$): 4,03 (s, 7- oder 17-OMe), 4,02 (s, 7- oder 17-OMe), 3,61 (s, 14-OMe), 3,25 (q, 3x $CH_2$, Triethylammonium-$CH_2$), 2,49 (s, N-Me), 2,03 (s, 16-Me), 1,91(s, 6-Me), 1,34 (t, 3x $CH_3$, Triethylammonium-$CH_3$), 1,09 (d, 25-Me). - HPLC, Standardbedingungen: $R_t$ = 2,38 min.

Beispiel 11: N-Trifluoracetyl-21-cyano-Saframycin Mx 1

20 mg 21-Cyano-Saframycin Mx 1 werden in 2 ml Pyridin gelöst und unter Argon mit einem Ueberschuss an Trifluoressigsäureanhydrid versetzt. Für 30 Min wird gerührt, danach in eine kalte Natrium-hydrogencarbonat-Lösung gegossen und mit Essigester extrahiert. Der Essigsäureethylester wird getrocknet und eingedampft. Die Titelverbindung wird über RP-Mitteldruck-Chromatographie mit Methanol: Puffer pH 6,5 = 55:45 gereinigt.

HPLC (Säule: RP-18 Nucleosil, 5 µm, 10 cm; Laufmittel: Methanol: Puffer (pH 6,5) = 55:45, Fluss: 1,5 ml/min): $R_t$ = 5,57 Min (Saframycin Mx 3:3,12 Min). 1H-NMR ($CDCl_3$ + 5 % $CD_3OD$):$\delta$ = 3,91 ppm (s,7-OMe), 3,65 (s, 17-OMe), 3,57 (s, 14-OMe), 2,32 (s, N-Me), 2,09 (s, 16-Me), 1,83 (s, 6-Me), 0,63 (d, J = 6,9 Hz, 25-Me).-13C-NMR ($CDCl_3$ + 5 % $CD_3OD$): $\delta$ = 157,77 (q, J = 37,6 Hz, O = C-$CF_3$), 115,56 ppm (q, J = 286,0 Hz, $CF_3$-C = O); UV (Methanol): $\lambda_{max}$ = 266 nm (1g $\varepsilon$ = 4,32).

Beispiel 12: N-(3-Carboxypropionyl)-21-CN-Saframycin MX 1 (2)

20 mg N-(3-Carboxypropionyl)-Saframycin Mx 1 (Beispiel 13a) werden in 5 ml Phosphatpuffer-Lösung pH 6 aufgenommen, mit einem geringen Ueberschuss an Natrium-cyanid versetzt und für 10 Min gerührt. Die wässrige Lösung wird auf eine kleine, mit Wasser equilibrierte XAD-2-Säule aufgetragen. Mit zwei Säulenvolumina Wasser wird zur Entfernung des Salzes gewaschen. Die Titelverbindung wird mit Methanol eluiert.

Die physikochemischen Charakteristika sind die gleichen wie im Beispiel 5i) angegeben.

Beispiel 13: N-Acyl-Saframycin Mx 1-Derivate

Allgemeine Arbeitsvorschrift:

In einer Stickstoffatmosphäre werden 50 mg Saframycin Mx 1 in 3 ml Phosphatpuffer pH 7,5 gelöst und mit 3 ml Methanol verdünnt. Zu dieser Lösung werden 1-2 Moläquivalente des entsprechenden Acylierungsreagenses als Carbonsäure-Anhydrid gelöst in 5 ml Tetrahydrofuran zugetropft. Die Reaktionsmischung wird 30 Minuten gerührt. Gemäss HPLC (Standardbedingungen: HD-Sil® 18, 7 µm, Laufmittel Methanol:Phosphat-Puffer 0,06 M, pH 6,5 = 60:40, Fluss ml/min Retentionszeit $R_t$ = 3,03 min) ist je nach Acylierungsmittel das Ausgangsmaterial Mx 1 nach 5-20 Minuten vollständig verbraucht. Der pH der Reaktionslösung wird ständig überwacht und kontinuierlich mit 1N NaOH auf pH 7,5 gehalten. Das gesamte Reaktionsgemisch wird nach Beendigung der Umsetzung auf eine XAD-2®-Säule (2,5 cm x 10 cm) aufgetragen. Die Säule wird mit zwei Bettvolumina Wasser gespült. Danach wird das gewünschte Produkt mit Methanol eluiert. Das Methanol wird im Vakuum abdestilliert und das acylierte Saframycin durch Abfiltrieren vom wässrigen Rückstand oder durch Gefriertrocknung isoliert.

Wenn Luftsauerstoff zur Lösung tritt, wird nach dieser Vorschrift neben N-Acyl-Saframycin Mx 1 gleichzeitig auch N-Acyl-Saframycin Mx 1 BC (das Bis-chinon) gebildet. Die beiden Verbindungen werden durch semipräparative HPLC an reversed phase Kieselgel (HD-Sil® 18) getrennt.

Nach dieser Arbeitsvorschrift wurden die folgenden Verbindungen hergestellt:

a) N-3-Carboxypropionyl-Saframycin Mx 1 (1), hergestellt aus Bernsteinsäureanhydrid:

$^1$H-NMR in $CD_3OD$: $\delta$ = 4,00 ppm (s, 7-OMe), 3,72 (s, 17-OMe), 3,66 (s, 14-OMe), 2,60-2,30 (m, 2x Succinyl-$CH_2$), 2,48 (s, N-Me), 2,25 (s, 16-Me), 1,89 (s, 6-Me), 0,78 (d, 25-Me). - FAB-MS des Triethylammoniumsalzes: Gef. 668,2717, für $C_{33}H_{40}N_4O_{11}$ ber. 668,2693, $(M-H_2O-H)^-$ des Bishydrochinons. - HPLC, Standardbedingungen: $R_t$ = 1,81 min.

b) N-(3-Carboxypropionyl)-Saframycin Mx 1 BC (1-B C):

$^1$H-NMR in $CD_3OD$: $\delta$ = 4,07 ppm (s, 17-OMe), 4,03 (s, 7-OMe), 3,62 (s, 14-OMe), 2,45 (s, N-Me), 2,33 (m, Succinyl-$CH_2$), 2,27 (m, Succinyl-$CH_2$), 1,99 (s, 16-Me), 1,90 (s, 6-Me), 1,18 (d, 25-Me). - HPLC, Standardbedingungen: $R_t$ = 2,24 min.

c) N-2-Carboxybenzoyl-Saframycin Mx 1 (2), hergestellt aus Phthalsäureanhydrid:

$^1$H-NMR in $CD_3OD$: $\delta$ = 7,82 ppm (m, 1H, Aromat), 7,47 (m, 2H, Aromat), 7,39 (m, 1H, Aromat), 4,02 (s, 7-OMe), 3,72 (s, 17-OMe), 3,65 (s, 14-OMe), 2,57 (s, N-Me), 2,10 (s, 16-Me), 1,84 (s, 6-Me), 0,94 (d, 25-Me). - FAB-MS: Gef. 716,2684, für $C_{37}H_{40}N_4O_{11}$ ber. 716,2693, $(M-H_2O)^-$. - HPLC, Standardbedingungen: $R_t$ = 1,75 min.

d) N-2-Carboxybenzoyl-Saframycin Mx 1 BC (2-BC):

$^1$H-NMR in $CD_{43}OD$: $\delta$ = 7,68 ppm (m, 1H, Aromat), 7,41 (m, 2H, Aromat), 7,34 (m, 1H, Aromat), 4,08 (s, 17-OMe), 3,98 (s, 7-OMe), 3,62 (s, 14-OMe), 2,45 (s, N-Me), 1,99 (s, 6-Me), 1,63 (s, 16-Me), 1,09 (d, 25-Me). - HPLC, Standardbedingungen: $R_t$ = 2,40 min.

e) N-3-Methoxycarbonylpropionyl-Saframycin Mx 1 (3), hergestellt aus Bernsteinsäuremonomethylester und Dicyclohexylcarbodiimid:

$^1$H-NMR in $CD_3OD$: $\delta$ = 3,99 ppm (s, 7-OMe), 3,74 (s, 17-OMe), 3,71 (s, Ester-Me), 3,69 (s, 14-OMe), 2,54 (s, N-Me), 2,54 (m, 2H, Succinyl-$CH_2$), 2,43 (m, 2H, Succinyl-$CH_2$), 2,23 (s, 16-Me), 1,90 (s, 6-Me), 0,9 (d,

25-Me). -FAB-MS: Gef. 682,2839, für $C_{34}H_{42}N_4O_{11}$ ber. 682,2850, $(M-H_2O)^-$. - HPLC, Standardbedingungen: $R_t$ = 3,15 min.

Beispiel 14: N-Sulfo-Saframycin Mx 1 und Mx 1 BC (4) und (4-BC)

80 mg Saframycin Mx 1 (1) werden in 3 ml Methanol und 3 ml Phosphatpuffer pH 7,8 (0,12 M) gelöst und mit der halben äquivalenten Menge an Pyridin-Schwefeltrioxid-Komplex versetzt. Mit 1N NaOH wird der pH auf den Ausgangswert 7,8 eingestellt. Danach wird nochmals die gleiche Menge Pyridin-Schwefeltrioxid zugesetzt. Nach 10 Min. ist die Reaktion beendet. Die Lösung wird im Oelpumpenvakuum eingeengt. Die Titelverbindungen werden über reversed phase-Mitteldruckchromatographie voneinander getrennt und als Triethylammoniumsalze isoliert.

a) N-Sulfo-Saframycin Mx 1 (4):

$^1$H-NMR ($CD_3OD$): δ = 4,04 ppm (s, 7-OMe), 3,76 (s, 17-OMe), 3,68 (s, 14-OMe), 3,25 (q, 3x $CH_2$, Triethylammonium-$CH_2$), 2,67 (s, N-Me), 2,31 (s, 16-Me), 1,89 (s, 6-Me), 1,34 (t, 3x $CH_3$, Triethylammonium-$CH_3$), 0,82 (d, 25-Me). - HPLC, Standardbedingungen: $R_t$ = 2,23 min.

b) N-Sulfo-Saframycin Mx 1 BC (4-BC):

$^1$H-NMR ($CD_3OD$): δ = 4,04 ppm (s, 7-oder 17-OMe), 4,03 (s, 7- oder 17-OMe), 3,62 (s, 14-OMe), 3,25 (q, 3x $CH_2$, Triethylammonium-$CH_2$), 2,50 (s, N-Me), 2,04 (s, 16-Me), 1,91 (s, 6-Me), 1,35 (t, 3x $CH_3$, Triethylammonium-$CH_3$), 1,13 (d, 25-Me). - FAB-MS: Gef. 798, 2322, für $C_{36}H_{40}N_5SO_{14}$) ber. 798,2292 (M + Nitrobenzylalkohol aus der Matrix)$^-$. - HPLC, Standardbedingungen: $R_t$ = 3,19 min.

Beispiel 15: Cytotoxizitätten der Derivat gegen Mäusefibroblasten L 929

Methanolische Lösungen der getesteten Verbindungen werden in Titerplatten mit 96 Näpfen $3^n$-fach mit DME-Medium (Dulbecco-Modifikation des Eagle-Medium mit 10 % fötalem Kälberserum) folgendermassen verdünnt:

| n | 2 | 3 | 4 | 5 | 6 | 7 | 8 | 9 | 10 | 11 | 12 |
|---|---|---|---|---|---|---|---|---|---|---|---|
| Konz. | 37000 | 12000 | 4100 | 1400 | 460 | 150 | 51 | 17 | 5,6 | 1,9 | 0,6 ng/ml |

Zu 60 µl der Verdünnungen wurden 120 µl einer durch Trypsinisieren erhaltenen Zellsuspension von Mäusefibroblasten der Linie L 929 gegeben, die 25000 Zellen/ml DME-Medium enthält (L 929: als Zellrasen wachsende, permanente Zellkultur). Die Titerplatten werden in einem befeuchteten Brutschrank bei 37° C und 10 % $CO_2$ für 5 Tage inkubiert. Danach wird das Wachstum der Mäusezellen unter dem Mikroskop beurteilt und die minimalen Hemmkonzentrationsstufen (MIC in ng/ml) der Verbin dungen bestimmt (Wachstum < 50 %):

| Verbindungen der Formel I | minimale Hemmkonzentration MIC (ng/ml) 4 Tage |
|---|---|
| 1 (Vergleich) | < 0,6 |
| 2-BC | 51 |
| 3 | 150 |
| 3-BC | 150 |
| 4 + 4-BC | 50 |
| 5-BC | 150 |
| 6 + 6-BC | 150 |
| 7 | 4100 |
| 7-BC | 4100 |
| 8 | 4100 |
| 9 | 51 |
| 10-BC | 150 |
| 11 | 150 |
| 11-BC | 150 |
| 12 | 4100 |
| 13 | 150 |
| 13-BC | 150 |
| 14 | 4100 |
| 14-BC | 4100 |

| Verbindungen der Formel IIb | minimale Hemmkonzentration MIC (ng/ml) nach 4 Tagen |
|---|---|
| 1 | 4100 |
| 1-BC | 4100 |
| 2 | 4100 |
| 3 | 150 |

| Verbindungen der Formel IIb | minimale Hemmkonzentration MIC (ng/ml) nach 4 Tagen |
|---|---|
| 1 | 4100 |
| 1-BC | 4100 |
| 2 | 4100 |
| 3 | 150 |

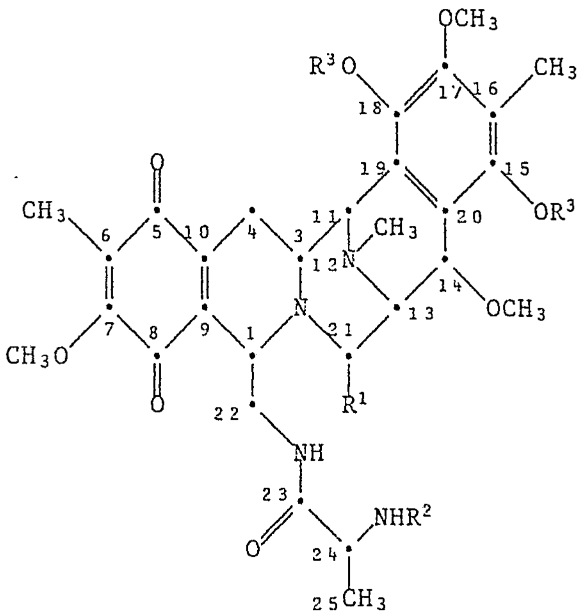

Verbindungen der Formel I

Nr.

1: $R^1$ = OH, $R^2$ = H, $R^3$ = H

2: $R^1$ = CN, $R^2$ = H, $R^3$ = H

3: $R^1$ = CN, $R^2$ = Acetyl, $R^3$ = H

4: $R^1$ = CN, $R^2$ = Caproyl, $R^3$ = H

5: $R^1$ = CN, $R^2$ = Benzoyl, $R^3$ = H

6: $R^1$ = CN, $R^2$ = Pivaloyl, $R^3$ = H

7: $R^1$ = CN, $R^2$ = Succinyl, $R^3$ = H

8: $R^1$ = CN, $R^2$ = β-Aspartyl-N(Z)-O(Bn), $R^3$ = H

9: $R^1$ = CN, $R^2$ = Formyl, $R^3$ = H

10: $R^1$ = CN, $R^2$ = -C(=O)-NHPhe, $R^3$ = H

11: $R^1$ = CN, $R^2$ = -C(=S)-NHPhe, $R^3$ = H

12: $R^1$ = CN, $R^2$ = Acetyl, $R^3$ = Acetyl

13: $R^1$ = CN, $R^2$ = Tosyl, $R^3$ = H

14: $R^1$ = CN, $R^2$ = Sulfo, $R^3$ = H

Verbindungen der Formel IIb

Nr.

1: $R^1$ = OH, $R^2$ = 3-Carboxypropionyl

2: $R^1$ = OH, $R^2$ = 2-Carboxybenzoyl

3: $R^1$ = OH, $R^2$ = 3-Methoxycarbonylpropionyl

4: $R^1$ = OH, $R^2$ = Sulfo

Beispiel 16: Pharmazeutisches Präparat zur parenteralen Verabreichung

Je 5 ml einer sterilen wässrigen Lösung von 1 % 21-Cyano-Saframycin Mx 1 (als Formiat) werden unter aseptischen Bedingungen in 5 ml-Ampullen oder Vials eingefüllt und gefriergetrocknet. Die Ampullen bzw. Vials werden unter Stickstoff verschlossen und geprüft.

Lösungen der acylierten und sulfonylierten 21-Cyano- und 21-Hydroxy-Saframycin Mx 1 und Mx 1 BC-Derivate, werden auf gleiche Art und Weise verarbeitet.

**Patentansprüche**

1. Verbindungen der Formel

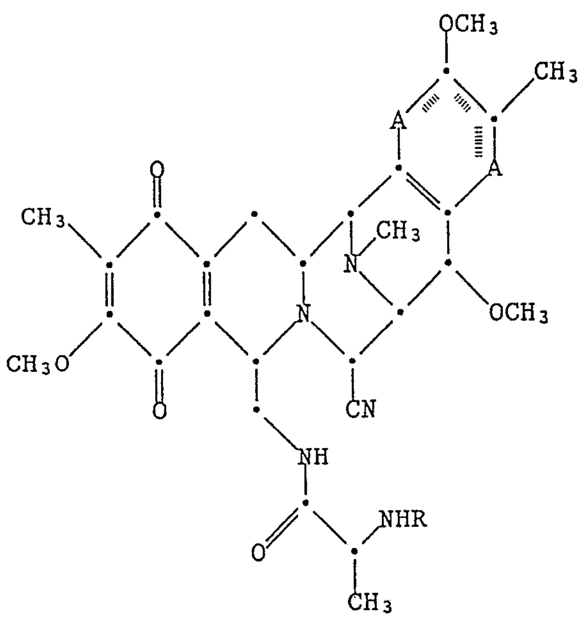

(I),

worin R Wasserstoff, Acyl, Sulfonyl, Sulfo oder Phospho und A C=O oder gegebenenfalls acyliertes C-OH bedeuten, wobei die gestrichelte Linie eine C=C-Doppelbindung an der Stelle der mittleren Bindung, wenn A C=O ist, oder an der Stelle der beiden äusseren Bindungen, wenn A gegebenenfalls

acyliertes C-OH ist, symbolisiert, und Salze solcher Verbindungen.

2. Verbindungen gemäss Anspruch 1, die die räumliche Struktur gemäss Formel Ia

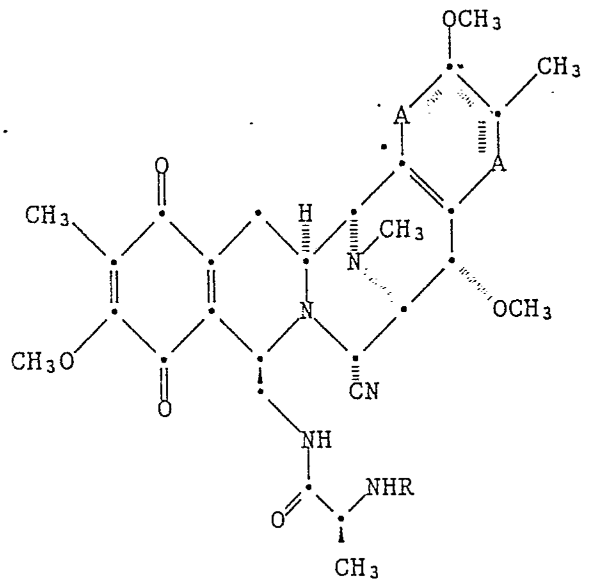

(Ia)

aufweisen.

3. Verbindungen der Formel I gemäss Anspruch 1 oder 2, worin R Wasserstoff, die Acylgruppe einer Carbonsäure, eines Kohlensäurehalbesters, einer Carbaminsäure oder einer Thiocarbaminsäure mit bis zu 20 Kohlenstoffatomen, der Acylrest eines Polypeptids aus 2-200 der üblichen in der Natur vorkommenden Aminosäuren, der Rest einer Sulfonsäure mit bis zu 20 Kohlenstoffatomen, unsubstituiertes oder substituiertes Aminosulfonyl, Sulfo oder Phospho, und A C=O oder gegebenenfalls acyliertes C-OH bedeuten, worin C-OH durch eine Carbonsäure mit bis zu 20 Kohlenstoffatomen acyliert sein kann, wobei die gestrichelte Linie eine C=C-Doppelbindung an der Stelle der mittleren Bindung, wenn A C=O ist, oder an der Stelle der beiden äusseren Bindungen, wenn A gegebenenfalls acyliertes C-OH ist, symbolisiert, und Salze solcher Verbindungen.

4. Verbindungen der Formel I gemäss Anspruch 1 oder 2, worin R Wasserstoff, $C_1$-$C_{20}$-Alkanoyl, durch Hydroxy, verethertes oder verestertes Hydroxy, Amino, acyliertes Amino, Carboxy, amidiertes oder verestertes Carboxy, Oxo und/oder Halogen substituiertes $C_2$-$C_7$-Alkanoyl, Aroyl, $C_1$-$C_7$-Alkoxycarbonyl, $C_1$-$C_7$-Alkylaminocarbonyl, $C_1$-$C_7$-Alkylaminothiocarbonyl, Arylaminocarbonyl, Arylaminothiocarbonyl, der Acylrest eines Polypeptids aus 2-200 der üblichen in der Natur vorkommenden Aminosäuren, $C_1$-$C_7$-Alkansulfonyl, Arylsulfonyl, Aminosulfonyl, $C_1$-$C_7$-Alkyl aminosulfonyl, Di($C_1$-$C_7$-alkyl)aminosulfonyl, Aryl-$C_1$-$C_4$-alkylaminosulfonyl, cyclisches fünf-oder sechsgliedriges Aminosulfonyl, worin der Cyclus ein bis zwei Stickstoffatome und gewünschtenfalls ein Sauerstoff- oder Schwefelatom enthält, Sulfo oder Phospho, und A C=O oder gegebenenfalls acyliertes C-OH, worin C-OH durch $C_1$-$C_{20}$-Alkanoyl, durch hydroxy, verethertes oder verestertes Hydroxy, Amino, acyliertes Amino, Carboxy, amidiertes oder verestertes Carboxy, Oxo und/oder Halogen substituiertes $C_2$-$C_7$-Alkanoyl oder Aroyl acyliert sein kann, bedeuten, wobei die gestrichelte Linie die obengenannte Bedeutung hat, und Salze solcher Verbindungen.

5. Verbindungen der Formel I gemäss Anspruch 1 oder 2, worin R Wasserstoff, $C_1$-$C_7$-Alkanoyl, durch gegebenenfalls acyliertes Amino und/oder verestertes Carboxy substituiertes $C_2$-$C_7$-Alkanoyl, Aroyl, Arylaminocarbonyl, Arylaminothiocarbonyl, Arylsulfonyl, Sulfo oder Phosphor, und A C=O oder gegebenenfalls acyliertes C-OH bedeuten, worin C-OH durch $C_1$-$C_7$-Alkanoyl oder durch Carboxy oder verestertes Carboxy substituiertes $C_2$-$C_7$-Alkanoyl acyliert sein kann, wobei die gestrichelte Linie die obengenannte Bedeutung hat, und pharmazeutische verwendbare Salze solcher Verbindungen.

6. Verbindungen der Formel I gemäss Anspruch 1 oder 2, worin R Wasserstoff, $C_1$-$C_7$-Alkanoyl, durch Carboxy, $C_1$-$C_4$-Alkoxycarbonyl oder Benzyloxycarbonyl und/oder Amino oder Benzyloxycarbonylamino substituiertes $C_2$-$C_7$-Alkanoyl, Phenylaminocarbonyl, Phenylaminothiocarbonyl, p-Toluolsulfonyl oder Sulfo, und A C=O oder gegebenenfalls acetyliertes C-OH bedeuten, wobei die gestrichelte Linie die obengenannte Bedeutung hat, und pharmazeutisch verwendbare Salze solcher Verbindungen.

7. Die Verbindung der Formel I gemäss Anspruch 1 oder 2, worin R Wasserstoff und A C-OH bedeuten, wobei die gestrichelte Linie die obengenannte Bedeutung hat, und pharmazeutische verwendbare Salze dieser Verbindung.

8. Die Verbindung der Formel I gemäss Anspruch 1 oder 2, worin R Formyl und A C-OH bedeuten,

wobei die gestrichelte Linie die obengennante Bedeutung hat, und pharmaceutisch verwendbare Salze dieser Verbindung.

9. Die Verbindung der Formel I gemäss Anspruch 1 oder 2, worin R n-Hexanoyl und A C-OH bedeuten, wobei die gestrichelte Linie die obengenannte Bedeutung hat, und pharmazeutisch verwendbare Salze dieser Verbindung.

10. Die Verbindung der Formel I gemäss Anspruch 1 oder 2, worin R 2,2-Dimethylpropionyl und A C-OH bedeuten, wobei die gestrichelte Linie die obengenannte Bedeutung hat, und pharmazeutisch verwendbare Salze dieser Verbindung.

11. Verfahren zur Herstellung von Verbindungen der Formel I gemäss Anspruch 1 oder 2, dadurch gekennzeichnet, dass man in einer Verbindung der Formel II

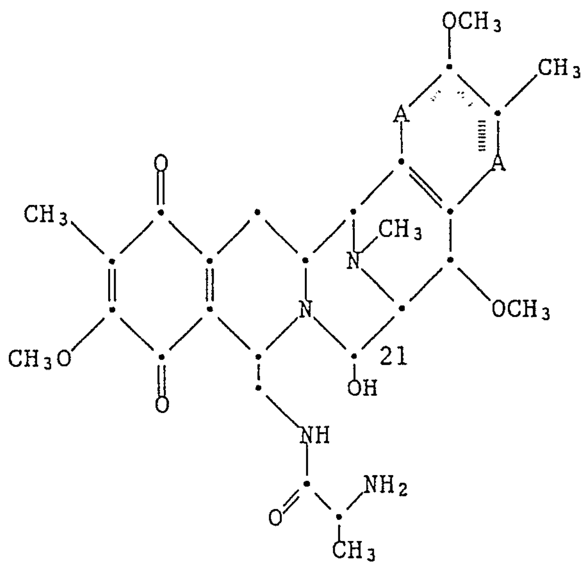

(II),

worin A C=O oder C-OH bedeuten, in beliebiger Reihenfolge die Hydroxygruppe am C-Atom 21 durch Cyanidierung in die Cyanogruppe und die $NH_2$-Gruppe durch Acylierung oder Sulfonierung in die NHR-Gruppe, worin R die unter Formel I angegebenen Bedeutungen hat, überführt, oder, zur Herstellung von Verbindungen der Formel I, worin R Wasserstoff ist, in einer Verbindung der Formel II die Hydroxygruppe am C-Atom 21 durch Cyanidierung in die Cyanogruppe überführt, und, wenn erwünscht, eine erfindungsgemässe Verbindung in eine andere erfindungs gemässe Verbindung überführt und/oder ein erhältliches Salz in die freie Verbindung oder ein anderes Salz oder eine erhältliche freie Verbindung in ein Salz überführt.

12. Die nach dem Verfahren gemäss Anspruch 11 erhältlichen antibiotisch wirksamen Verbindungen.

13. Pharmazeutische Präparate enthaltend Verbindungen der Formel I oder pharmazeutisch verwendbare Salze davon gemäss Anspruch 1 oder 2 im Gemisch mit pharmazeutisch verwendbaren Trägerstoffen.

14. Verwendung von Verbindungen der Formel I oder von pharmazeutisch verwendbaren Salzen davon gemäss Anspruch 1 oder 2 zur Herstellung von pharmazeutischen Präparaten.

15. Verbindungen der Formel

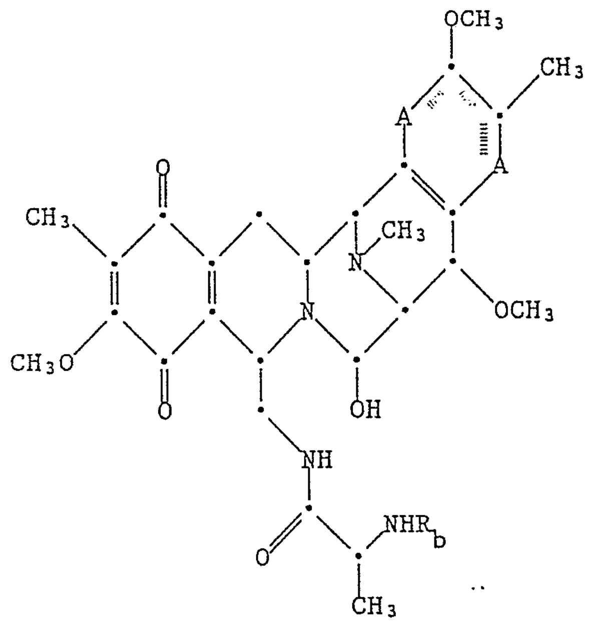

(IIb),

worin $R_b$ durch Carboxy oder verestertes Carboxy substituiertes Acyl, Sulfo oder Phospho und A C=O oder gegebenenfalls acyliertes C-OH bedeuten, wobei die gestrichelte Linie eine C=C-Doppelbindung an der Stelle der mittleren Bindung, wenn A C=O ist, oder an der Stelle der beiden äusseren Bindungen, wenn A gegebenenfalls acyliertes C-OH ist, symbolisiert, und Salze solcher Verbindungen.

16. Verbindungen gemäss Anspruch 15, die die räumliche Struktur gemäss Formel $II_b$

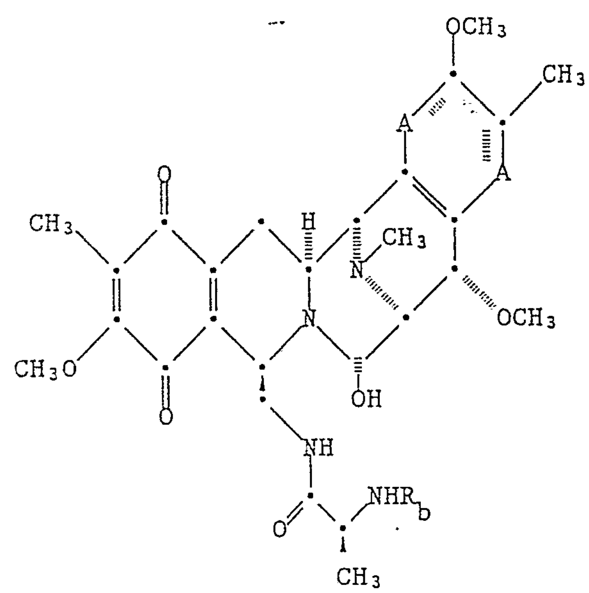

(IIb)

aufweisen.

17. Verbindungen der Formel IIb gemäss Anspruch 15 oder 16, worin $R_b$ durch Carboxy, $C_1$-$C_7$-Alkoxycarbonyl oder Aryl-$C_1$-$C_4$-alkoxycarbonyl und gegebenenfalls durch Amino, Acylamino, Hydroxy, $C_1$-$C_7$-Alkoxy und/oder $C_1$-$C_7$-Alkanoyloxy substituiertes $C_1$-$C_{20}$-Alkanoyl, $C_1$-$C_7$-Alkenoyl, Aroyl mit bis zu 20 C-Atomen oder Heteroaroyl mit bis zu 20 C-Atomen und 1 bis 2 Heteroatomen, Sulfo oder Phospho, und A C=O oder gegebenenfalls acyliertes C-OH bedeuten, wobei die gestrichelte Linie die obengenannte Bedeutung hat, und Salze solcher Verbindungen.

18. Verbindungen der Formel IIb gemäss Anspruch 15 oder 16, worin $R_b$ Carboxy-$C_1$-$C_7$-alkanoyl, $C_1$-$C_7$-Alkoxycarbonyl-$C_1$-$C_7$-alkanoyl, Aryl-$C_1$-$C_4$-alkoxycarbonyl-$C_1$-$C_7$-alkanoyl, Carboxy-aroyl, $C_1$-$C_7$-Alkoxycarbonyl-aroyl, Amino-carboxy-$C_1$-$C_7$-alkanoyl, Acylamino-$C_1$-$C_7$-alkoxycarbonyl-$C_1$-$C_7$-al-

kanoyl, Acylamino-aryl-$C_1$-$C_4$-alkoxycarbonyl-$C_1$-$C_7$-alkanoyl, Hydroxy-carbonyl-$C_1$-$C_7$-alkanoyl, Acyloxy-$C_1$-$C_7$-Alkoxycarbonyl-$C_1$-$C_7$-alkanoyl, Sulfo oder Phospho, und A C=O oder gegebenenfalls acyliertes C-OH bedeuten, worin C-OH durch $C_1$-$C_7$-Alkanoyl, Carboxy-$C_1$-$C_7$-alkanoyl, $C_1$-$C_7$-Alkoxycarbonyl-$C_1$-$C_7$-alkanoyl, Carboxy aroyl oder $C_1$-$C_7$-Alkoxycarbonyl-aroyl substituiert sein kann, wobei die gestrichelte Linie die obengenannte Bedeutung hat, und Salze solcher Verbindungen.

19. Verbindungen der Formel IIb gemäss Anspruch 15 oder 16, worin $R_b$ Carboxy-$C_1$-$C_7$-alkanoyl, $C_1$-$C_7$-Alkoxycarbonyl-$C_1$-$C_7$-alkanoyl, Carboxy-aroyl, $C_1$-$C_7$-Alkoxycarbonyl-aroyl, Amino-carboxy-$C_1$-$C_7$-alkanoyl, Sulfo oder Phospho, und A C=O oder gegebenenfalls acyliertes C-OH bedeuten, worin C-OH durch $C_1$-$C_7$-Alkanoyl, Carboxy-$C_1$-$C_7$-alkanoyl oder $C_1$-$C_7$-Alkoxycarbonyl-$C_1$-$C_7$-alkanoyl substituiert sein kann, wobei die gestrichelte Linie die obengenannte Bedeutung hat, und Salze dieser Verbindungen.

20. Verbindungen der Formel IIb gemäss Anspruch 15 oder 16, worin $R_b$ Carboxy-$C_1$-$C_7$-alkanoyl, $C_1$-$C_7$-Alkoxycarbonyl-$C_1$-$C_7$-alkanoyl, Carboxybenzoyl oder Sulfo, und A C=O oder gegebenenfalls acetyliertes C-OH bedeuten, wobei die gestrichelte Linie die obengenannte Bedeutung hat, und pharmazeutisch verwendbare Salze solcher Verbindungen.

21. Die Verbindung der Formel $II_b$ gemäss Anspruch 15 oder 16, worin $R_b$ 3-Carboxypropionyl und A C-OH bedeuten, wobei die gestrichelte Linie die obengenannte Bedeutung hat, und pharmazeutisch verwendbare Salze dieser Verbindung.

22. Die Verbindung der Formel $II_b$ gemäss Anspruch 15 oder 16, worin $R_b$ 2-Carboxybenzoyl und A C-OH bedeuten, wobei die gestrichelte Linie die obengenannte Bedeutung hat, und pharmazeutisch verwendbare Salze dieser Verbindung.

23. Verfahren zur Herstellung von Verbindungen der Formel IIb gemäss Anspruch 15 oder 16, dadurch gekennzeichnet, dass man eine Verbindung der Formel II

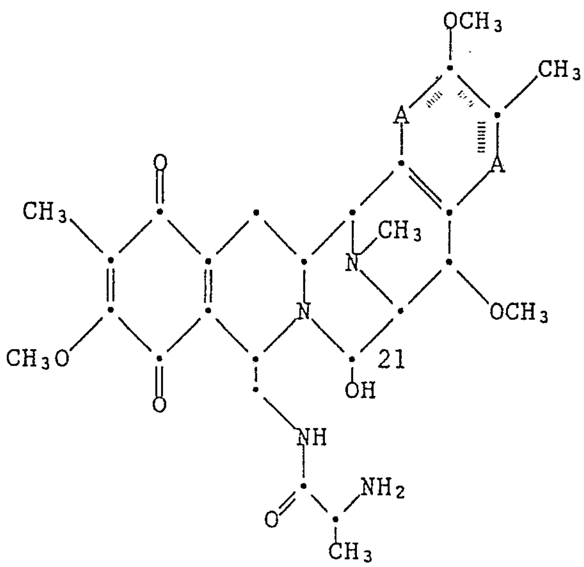

(II)

worin A C=O oder C-OH bedeuten, acyliert und, wenn erwünscht, eine erfindungsgemässe Verbindung in eine andere erfindungsgemässe Verbindung überführt und/oder ein erhältliches Salz in die freie Verbindung oder ein anderes Salz oder eine erhältliche freie Verbindung in ein Salz überführt.

24. Die nach dem Verfahren gemäss Anspruch 23 erhältlichen antibiotisch wirksamen Verbindungen.

25. Pharmazeutische Präparate enthaltend Verbindungen der Formel IIb oder pharmazeutisch verwendbare Salze davon gemäss Anspruch 15 oder 16 im Gemisch mit pharmazeutisch verwendbare Trägerstoffen.

26. Verwendung von Verbindungen der Formel IIb oder von pharmazeutisch verwendbaren Salzen davon gemäss Anspruch 15 oder 16 zur Herstellung von pharmazeutischen Präparaten.

**Patentansprüche für folgende Vertragsstaaten : ES, GR**

1. Verfahren zur Herstellung von Verbindungen der Formel

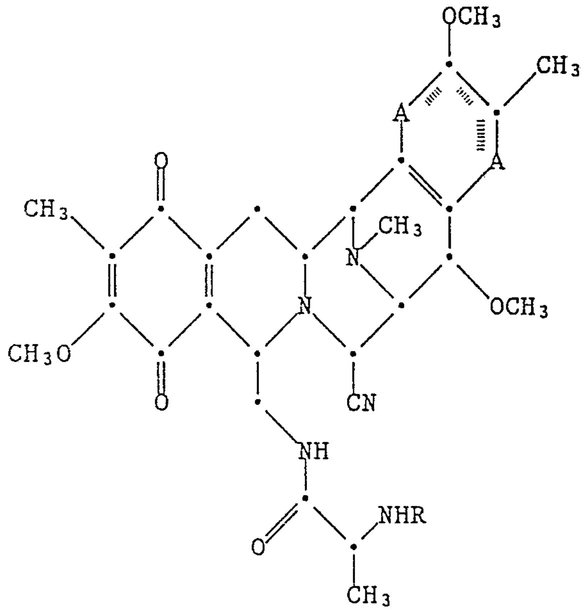

(I),

worin R Wasserstoff, Acyl, Sulfonyl, Sulfo oder Phospho und A C=O oder gegebenenfalls acyliertes C-OH bedeuten, wobei die gestrichelte Linie eine C=C-Doppelbindung an der Stelle der mittleren Bindung, wenn A C=O ist, oder an der Stelle der beiden äusseren Bindungen, wenn A gegebenenfalls acyliertes C-OH ist, symbolisiert, und Salzen solcher Verbindungen, dadurch gekennzeichnet, dass man in einer Verbindung der Formel II

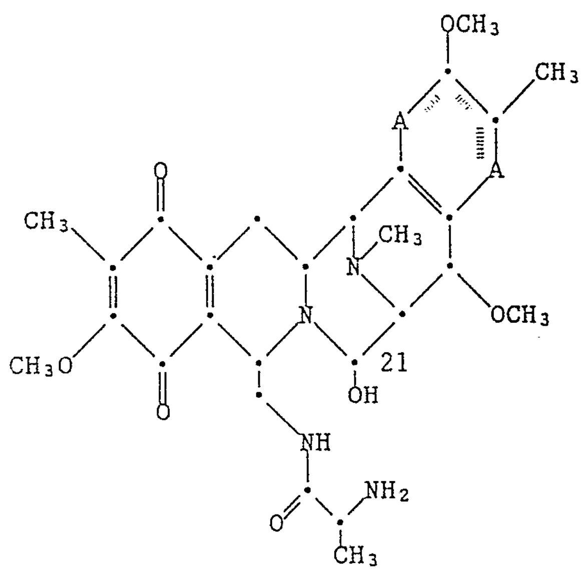

(II),

worin A C=O oder C-OH bedeuten, in beliebiger Reihenfolge die Hydroxygruppe am C-Atom 21 durch Cyanidierung in die Cyanogruppe und die $NH_2$-Gruppe durch Acylierung oder Sulfonierung in die NHR-Gruppe, überführt, oder, zur Herstellung von Verbindungen der Formel I, worin R Wasserstoff ist, in einer Verbindung der Formel II die Hydroxygruppe am C-Atom 21 durch Cyanidierung in die Cyanogruppe überführt, und, wenn erwünscht, eine erfindungsgemässe Verbindung in eine andere erfindungsgemässe Verbindung überführt und/oder ein erhältliches Salz in die freie Verbindung oder ein anderes Salz oder eine erhältliche freie Verbindung in ein Salz überführt.

2. Verfahren gemäss Anspruch 1 zur Herstellung von Verbindungen, die die räumliche Struktur gemäss Formel Ia

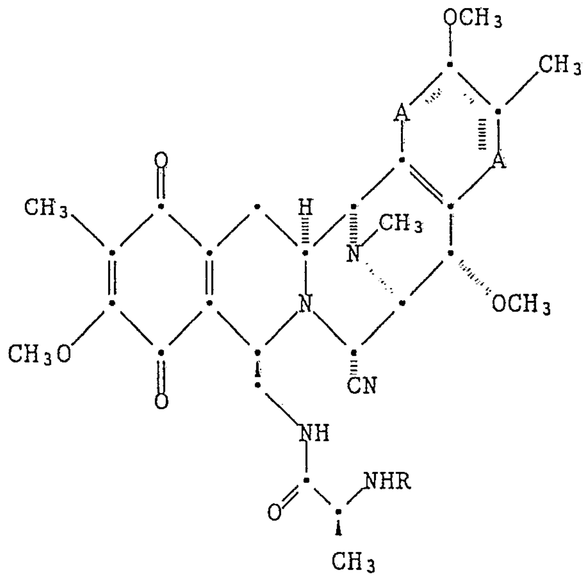

(Ia)

aufweisen.

3. Verfahren zur Herstellung von Verbindungen der Formel I gemäss Anspruch 1 oder 2, worin R Wasserstoff, die Acylgruppe einer Carbonsäure, eines Kohlensäurehalbesters, einer Carbaminsäure oder einer Thiocarbaminsäure mit bis zu 20 Kohlenstoffatomen, der Acylrest eines Polypeptids aus 2-200 der üblichen in der Natur vorkommenden Aminosäuren, der Rest einer Sulfonsäure mit bis zu 20 Kohlenstoffatomen, unsubstituiertes oder substituiertes Aminosulfonyl, Sulfo oder Phospho, und A C=O oder gegebenenfalls acyliertes C-OH bedeuten, worin C-OH durch eine Carbonsäure mit bis zu 20 Kohlenstoffatomen acyliert sein kann, wobei die gestrichelte Linie eine C=C-Doppelbindung an der Stelle der mittleren Bindung, wenn A C=O ist, oder an der Stelle der beiden äusseren Bindungen, wenn A gegebenenfalls acyliertes C-OH ist, symbolisiert, und Salzen solcher Verbindungen.

4. Verfahren zur Herstellung von Verbindungen der Formel I gemäss Anspruch 1 oder 2, worin R Wasserstoff, $C_1$-$C_{20}$-Alkanoyl, durch Hydroxy, verethertes oder verestertes Hydroxy, Amino, acyliertes Amino, Carboxy, amidiertes oder verestertes Carboxy, Oxo und/oder Halogen substituiertes $C_2$-$C_7$-Alkanoyl, Aroyl, $C_1$-$C_7$-Alkoxycarbonyl, $C_1$-$C_7$-Alkylaminocarbonyl, $C_1$-$C_7$-Alkylaminothiocarbonyl, Arylaminocarbonyl, Arylaminothiocarbonyl, der Acylrest eines Polypeptids aus 2-200 der üblichen in der Natur vorkommenden Aminosäuren, $C_1$-$C_7$-Alkansulfonyl, Arylsulfonyl, Aminosulfonyl, $C_1$-$C_7$-Alkylaminosulfonyl, Di($C_1$-$C_7$-alkyl)aminosulfonyl, Aryl-$C_1$-$C_4$-alkylaminosulfonyl, cyclisches fünf-oder sechsgliedriges Aminosulfonyl, worin der Cyclus ein bis zwei Stickstoffatome und gewünschtenfalls ein Sauerstoff- oder Schwefelatom enthält, Sulfo oder Phospho, und A C=O oder gegebenenfalls acyliertes C-OH, worin C-OH durch $C_1$-$C_{20}$-Alkanoyl, durch Hydroxy, verethertes oder verestertes Hydroxy, Amino, acyliertes Amino, Carboxy, amidiertes oer verestertes Carboxy, Oxo und/oder Halogen substituiertes $C_2$-$C_7$-Alkanoyl oder Aroyl acyliert sein kann, bedeuten, wobei die gestrichelte Linie die obengenannte Bedeutung hat, und Salzen solcher Verbindungen.

5. Verfahren zur Herstellung von Verbindungen der Formel I gemäss Anspruch 1 oder 2, worin R Wasserstoff, $C_1$-$C_7$-Alkanoyl, durch gegebenenfalls acyliertes Amino und/oder verestertes Carboxy substituiertes $C_2$-$C_7$-Alkanoyl, Aroyl, Arylaminocarbonyl, Arylaminothiocarbonyl, Arylsulfonyl, Sulfo oder Phospho, und A C=O oder gegebenenfalls acyliertes C-OH bedeuten, worin C-OH durch $C_1$-$C_7$-Alkanoyl oder durch Carboxy oder verestertes Carboxy substituiertes $C_2$-$C_7$-Alkanoyl acyliert sein kann, wobei die gestrichelte Linie die obengenannte Bedeutung hat, und pharmazeutisch verwendbaren Salzen solcher Verbindungen.

6. Verfahren zur Herstellung von Verbindungen der Formel I gemäss Anspruch 1 oder 2, worin R Wasserstoff, $C_1$-$C_7$-Alkanoyl, durch Carboxy, $C_1$-$C_4$-Alkoxycarbonyl oder Benzyloxycarbonyl und/oder Amino oder Benzyloxycarbonylamino substituiertes $C_2$-$C_7$-Alkanoyl, Phenylaminocarbonyl, Phenylaminothiocarbonyl, p-Toluolsulfonyl oder Sulfo, und A C=O oder gegebenenfalls acetyliertes C-OH bedeuten, wobei die gestrichelte Linie die obengenannte Bedeutung hat, und pharmazeutisch verwendbaren Salzen solcher Verbindungen.

7. Verfahren zur Herstellung der Verbindung der Formel I gemäss Anspruch 1 oder 2, worin R Wasserstoff und A C-OH bedeuten, wobei die gestrichelte Linie die obengenannte Bedeutung hat, und pharmazeutisch verwendbaren Salzen dieser Verbindung.

8. Verfahren zur Herstellung der Verbindung der Formel I gemäss Anspruch 1 oder 2, worin R Formyl

und A C-OH bedeuten, wobei die gestrichelte Linie die obengenannte Bedeutung hat, und pharmazeutisch verwendbaren Salzen dieser Verbindung.

9. Verfahren zur Herstellung der Verbindung der Formel I gemäss Anspruch 1 oder 2, worin R n-Hexanoyl und A C-OH bedeuten, wobei die gestrichelte Linie die obengenannte Bedeutung hat, und pharmazeutisch verwendbaren Salzen dieser Verbindung.

10. Verfahren zur Herstellung der Verbindung der Formel I gemäss Anspruch 1 oder 2, worin R 2,2-Dimethylpropionyl und A C-OH bedeuten, wobei die gestrichelte Linie die obengenannte Bedeutung hat, und pharmazeutisch verwendbaren Salzen dieser Verbindung.

11. Verfahren zur Herstellung von pharmazeutischen Präparaten enthaltend Verbindungen der Formel I oder pharmazeutisch verwendbaren Salzen davon gemäss Anspruch 1 oder 2 im Gemisch mit pharmazeutisch verwendbaren Trägerstoffen.

12. Verwendung von Verbindungen der Formel I oder von pharmazeutisch verwendbaren Salzen davon gemäss Anspruch 1 oder 2 zur Herstellung von pharmazeutischen Präparaten.

13. Verfahren zur Herstellung von Verbindungen der Formel

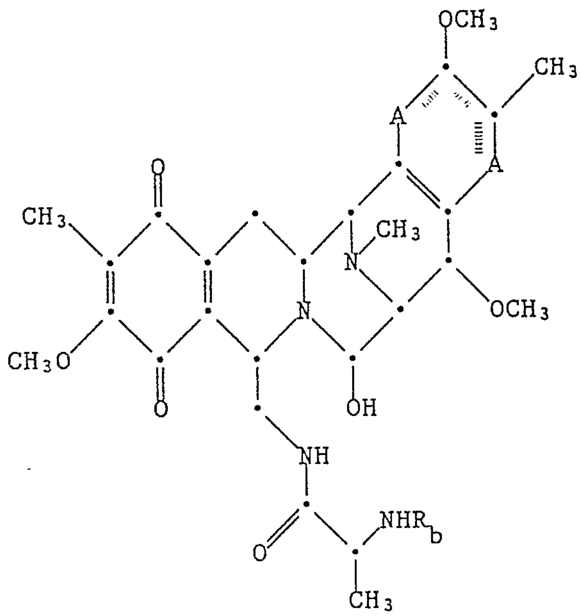

(IIb),

worin $R_b$ durch Carboxy oder verestertes Carboxy substituiertes Acyl, Sulfo oder Phospho und A C=O oder gegebenenfalls acyliertes C-OH bedeuten, wobei die gestrichelte Linie eine C=C-Doppelbindung an der Stelle der mittleren Bindung, wenn A C=O ist, oder an der Stelle der beiden äusseren Bindungen, wenn A gegebenenfalls acyliertes C-OH ist, symbolisiert, und Salzen solcher Verbindungen, dadurch gekennzeichnet, dass man eine Verbindung der Formel II

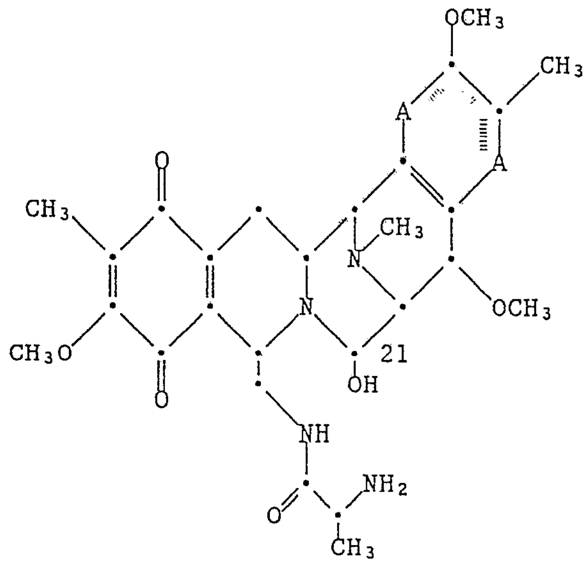

(II)

worin A C=O oder C-OH bedeuten, acyliert und, wenn erwünscht, eine erfindungsgemässe Verbindung in eine andere erfindungsgemässe Verbindung überführt und/oder ein erhältliches Salz in die freie Verbindung oder ein anderes Salz oder eine erhältliche freie Verbindung in ein Salz überführt.

14. Verfahren zur Herstellung von Verbindungen gemäss Anspruch 13, die die räumliche Struktur gemäss Formel $II_b$

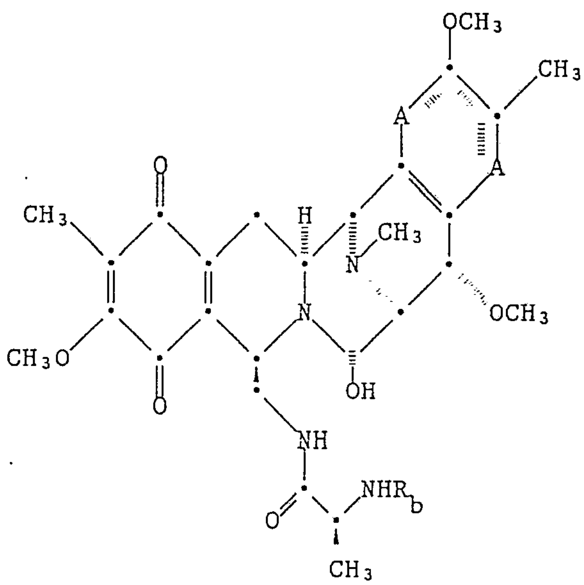

(IIb)

aufweisen.

15. Verfahren zur Herstellung von Verbindungen der Formel IIb gemäss Anspruch 13 oder 14, worin $R_b$ durch Carboxy, $C_1$-$C_7$-Alkoxycarbonyl oder Aryl-$C_1$-$C_4$-alkoxycarbonyl und gegebenenfalls durch Amino, Acylamino, Hydroxy, $C_1$-$C_7$-Alkoxy und/oder $C_1$-$C_7$-Alkanoyloxy substituiertes $C_1$-$C_{20}$-Alkanoyl, $C_1$-$C_7$-Alkenoyl, Aroyl mit bis zu 20 C-Atomen oder Heteroaroyl mit bis zu 20 C-Atomen und 1 bis 2 Heteroatomen, Sulfo oder Phospho, und A C=O oder gegebenenfalls acyliertes C-OH bedeuten, wobei die gestrichelte Linie die obengenannte Bedeutung hat, und Salzen solcher Verbindungen.

16. Verfahren zur Herstellung von Verbindungen der Formel IIb gemäss Anspruch 13 oder 14, worin $R_b$ Carboxy-$C_1$-$C_7$-alkanoyl, $C_1$-$C_7$-Alkoxycarbonyl-$C_1$-$C_7$-alkanoyl, Aryl-$C_1$-$C_4$-alkoxycarbonyl-$C_1$-$C_7$-alkanoyl, Carboxy-aroyl, $C_1$-$C_7$-Alkoxycarbonyl-aroyl, Amino-carboxy-$C_1$-$C_7$-alkanoyl, Acylamino-$C_1$-$C_7$-alkoxycarbonyl-$C_1$-$C_7$-alkanoyl, Acylamino-aryl-$C_1$-$C_4$-alkoxycarbonyl-$C_1$-$C_7$-alkanoyl, Hydroxy-carboxy-

$C_1$-$C_7$-alkanoyl, Acyloxy-$C_1$-$C_7$-Alkoxycarbonyl-$C_1$-$C_7$-alkanoyl, Sulfo oder Phospho, und A C=O oder gegebenenfalls acyliertes C-OH bedeuten, worin C-OH durch $C_1$-$C_7$-Alkanoyl, Carboxy-$C_1$-$C_7$-alkanoyl, $C_1$-$C_7$-Alkoxycarbonyl-$C_1$-$C_7$-alkanoyl, Carboxy-aroyl oder $C_1$-$C_7$-Alkoxycarbonyl-aroyl substituiert sein kann, wobei die gestrichelte Linie die obengenannte Bedeutung hat, und Salzen solcher Verbindungen.

17. Verfahren zur Herstellung von Verbindungen der Formel IIb gemäss Anspruch 13 oder 14, worin $R_b$ Carboxy-$C_1$-$C_7$-alkanoyl, $C_1$-$C_7$-Alkoxycarbonyl-$C_1$-$C_7$-alkanoyl, Carboxy-aroyl, $C_1$-$C_7$-Alkoxycarbonyl-aroyl, Amino-carboxy-$C_1$-$C_7$-alkanoyl, Sulfo oder Phospho, und A C=O oder gegebenenfalls acyliertes C-OH bedeuten, worin C-OH durch $C_1$-$C_7$-Alkanoyl, Carboxy-$C_1$-$C_7$-alkanoyl oder $C_1$-$C_7$-Alkoxycarbonyl-$C_1$-$C_7$-alkanoyl substituiert sein kann, wobei die gestrichelte Linie die obengenannte Bedeutung hat, und Salzen dieser Verbindungen.

18. Verfahren zur Herstellung von Verbindungen der Formel IIb gemäss Anspruch 13 oder 14, worin $R_b$ Carboxy-$C_1$-$C_7$-alkanoyl, $C_1$-$C_7$-Alkoxycarbonyl-$C_1$-$C_7$-alkanoyl, Carboxybenzoyl oder Sulfo, und A C=O oder gegebenenfalls acetyliertes C-OH bedeuten, wobei die gestrichelte Linie die obengenannte Bedeutung hat, und pharmazeutisch verwendbaren Salzen solcher Verbindungen.

19. Verfahren zur Herstellung der Verbindung der Formel $II_b$ gemäss Anspruch 13 oder 14, worin $R_b$ 3-Carboxypropionyl und A C-OH bedeuten, wobei die gestrichelte Linie die obengenannte Bedeutung hat, und pharmazeutisch verwendbaren Salzen dieser Verbindung.

20. Verfahren zur Herstellung der Verbindung der Formel $II_b$ gemäss Anspruch 13 oder 14, worin $R_b$ 2-Carboxybenzoyl und A C-OH bedeuten, wobei die gestrichelte Linie die obengenannte Bedeutung hat, und pharmazeutisch verwendbaren Salzen dieser Verbindung.

21. Verfahren zur Herstellung von pharmazeutischen Präparaten enthaltend Verbindungen der Formel IIb oder pharmazeutisch verwendbaren Salzen davon gemäss Anspruch 13 oder 14 im Gemisch mit pharmazeutisch verwendbaren Trägerstoffen.

22. Verwendung von Verbindungen der Formel IIb oder von pharmazeutisch verwendbaren Salzen davon gemäss Anspruch 13 oder 14 zur Herstellung von pharmazeutischen Präparaten.